(19) Europäisches Patentamt
European Patent Office
Office européen des brevets

(11) **EP 2 780 365 B1**

(12) **EUROPEAN PATENT SPECIFICATION**

(45) Date of publication and mention
of the grant of the patent:
**22.02.2017 Bulletin 2017/08**

(51) Int Cl.:
*C07K 14/79* (2006.01)    *A61K 38/40* (2006.01)
*A61P 31/16* (2006.01)

(21) Application number: **12813542.3**

(86) International application number:
**PCT/IT2012/000349**

(22) Date of filing: **16.11.2012**

(87) International publication number:
**WO 2013/072946 (23.05.2013 Gazette 2013/21)**

(54) **LACTOFERRIN DERIVED PEPTIDES FOR USE AS BROAD- SPECTRUM INHIBITORS OF INFLUENZA VIRUS INFECTION**

PEPTIDE AUS LACTOFERRIN ZUR VERWENDUNG ALS BREITSPEKTRUM-INHIBITOREN EINER INFLUENZAVIRUSINFEKTION

PEPTIDES DÉRIVÉS DE LACTOFERRINE POUR UTILISATION EN TANT QU'INHIBITEURS À LARGE SPECTRE D'UNE INFECTION PAR LE VIRUS DE LA GRIPPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2011 IT RM20110606**

(43) Date of publication of application:
**24.09.2014 Bulletin 2014/39**

(73) Proprietors:
• **Istituto Superiore di Sanità**
**00161 Roma (IT)**
• **Universita' Degli Studi "G. d'Annunzio"**
**Chieti-Pescara**
**66100 Chieti (IT)**

(72) Inventors:
• **SUPERTI, Fabiana E. D.**
**00161 Roma RM (IT)**
• **AGAMENNONE, Mariangela**
**66100 Chieti (IT)**
• **AMMENDOLIA, Maria Grazia**
**00161 Roma RM (IT)**
• **PIETRANTONI, Agostina**
**00161 Roma RM (IT)**
• **LANNUTTI, Fabio**
**66100 Chieti (IT)**

(74) Representative: **Gitto, Serena**
**Barzanò & Zanardo Roma S.p.A.**
**Via Piemonte, 26**
**00187 Roma (IT)**

(56) References cited:
**EP-A2- 0 643 074        WO-A1-2007/043900**
**US-A1- 2011 245 461      US-B2- 7 267 942**

• CHEN X ET AL: "Peptide of decreasing blood pressure of protein of acetes chinensis, preparing method and application", WPI / THOMSON,, vol. 2006, no. 24, 31 August 2005 (2005-08-31), XP002678776, -& CN 1 660 888 A (SHANDONG [CN] UNIV SHANDONG [CN]) 31 August 2005 (2005-08-31)
• AGOSTINA PIETRANTONI ET AL: "Bovine lactoferrin inhibits Influenza A virus induced programmed cell death in vitro", BIOMETALS, KLUWER ACADEMIC PUBLISHERS, BO, vol. 23, no. 3, 16 March 2010 (2010-03-16), pages 465-475, XP019813985, ISSN: 1572-8773 cited in the application

- **FRANCESCA BERLUTTI ET AL: "Antiviral properties of lactoferrin--a natural immunity molecule", MOLECULES, MOLECULAR DIVERSITY PRESERVATION INTERNATIONAL, BASEL, CH , vol. 16, no. 8 1 January 2011 (2011-01-01), pages 6992-7018, XP002678777, ISSN: 1420-3049, DOI: 10.3390/MOLECULES16086992 Retrieved from the Internet: URL:http://www.mdpi.com/1420-3049/16/8/699 2 [retrieved on 2011-08-16]**
- **KAWASAKI YOSHIHIRO ET AL: "INHIBITION BY KAPPA-CASEIN GLYCOMACROPEPTIDE AND LACTOFERRIN OF INFLUENZA VIRUS HEMAGGLUTINATION", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 57, no. 7, 1 January 1993 (1993-01-01), pages 1214-1215, XP009072230, ISSN: 0916-8451**
- **AMMENDOLIA MARIA GRAZIA ET AL: "Bovine lactoferrin-derived peptides as novel broad-spectrum inhibitors of influenza virus", PATHOGENS AND GLOBAL HEALTH, LEEDS [U.A.] : MANEY, GB, vol. 106, no. 1, 1 March 2012 (2012-03-01) , pages 12-19, XP008153056, ISSN: 2047-7724**
- **DATABASE USPTO PROTEINS [Online] 19 October 2007 'Sequence 106 from patent US 7267942.' Retrieved from EBI, accession no. USPOP:ABW50189 Database accession no. ABW50189**

Remarks:

The file contains technical information submitted after the application was filed and not included in this specification

**Description**

The present invention concerns lactoferrin derived peptides for use as broad-spectrum inhibitors of Influenza virus infection. Particularly, the present invention concerns lactoferrin C-lobe derived peptides, preferably bovine lactoferrin C-lobe derived peptides, for use as broad-spectrum inhibitors of Influenza virus infection able to inhibit hemagglutination and infection of cells by Influenza virus belonging to all major subtypes including H1 N1 and H3N2.

Influenza virus infections are a major risk to human safety and animal health worldwide. Viral Influenza disease represents a serious source of morbidity and mortality worldwide and a considerable cause of illness and death among people with immunodeficiency associated with aging or different clinical conditions. Control of Influenza is essentially based on vaccines and few antiviral drugs. While vaccines are the core measure for infection control, the immunization programs are not fully effective because of rapid virus antigenic drift, so that vaccine antigen composition needs to be updated annually based on global influenza surveillance. Efforts to Influenza prevention by vaccination are made difficult by the virus ability to rapidly mutate and recombine into antigenically new viral particles, sometimes leading to the emergence of a totally new virus.

Antiviral chemotherapy is based on two classes of drugs: amantadine and its derivative rimantadine (Dolin *et al.,* 1982) and neuraminidase inhibitors (oseltamivir and zanamivir) (Smith et al., 2011 aggiunto). Amantadine and derivatives prevent viral uncoating by blocking the ion channel function of the M2 protein of the Influenza virus. These drugs reduce the duration of symptoms of clinical influenza, but major side effects and the emergence of drug-resistant variants have been described (Hayden 2006; Fiore *et al.,* 2008). Consequently, amantadine and derivatives are no longer the first choice for Influenza therapy. Neuraminidase inhibitors prevent viral budding from infected cells by inhibiting the cleavage of host sialic acid residues and remain, at present, the primary treatment against Influenza. However, they have limited efficacy if administered late in infection and widespread use is likely to result in the emergence of resistant viral strains (Kiso *et al.,* 2004; Dharan *et al.,* 2009; Cheng *et al.,* 2010).

The availability of broad-spectrum antiviral drugs is an important asset in the fight against influenza. Particularly, a combination of different anti-Influenza drugs, each directed against a different viral target or different mode of action, would be expected to be more active in Influenza treatment and also in minimizing drug resistance. In fact, combination of amantadine and oseltamivir has been shown to decrease the emergence of drug-resistant Influenza A viruses (Ilyushina *et al.,* 2006). Although this has not been observed in all cases.

On the basis of the above it is therefore apparent the need to provide for new compounds against Influenza virus able to overcome the disadvantages of the known therapies.

New therapeutic strategies are therefore a universal public health priority. An attractive antiviral strategy is the blocking of Influenza virus entry into the host cell. This process is mediated by the viral hemagglutinin (HA), which is responsible for the binding of the virus to the target cell and, after virus uptake into endosomes, fusion of the virus with the cell membranes (Skehel and Wiley, 2000). The HA of Influenza is the major glycoprotein component of the viral envelope. HA is homotrimeric and is composed of two polypeptide segments, designated $HA_1$ and $HA_2$, attached to each other via a disulfide linkage (Wilson *et al.,* 1981). The $HA_1$ segments mediate HA attachment to the host cell surface by binding to sialic acid-containing cell surface glycans. After attachment, virions internalised by endocytoses undergoes an irreversible acid-induced structural rearrangement in which the highly hydrophobic amino terminus of $HA_2$ is exposed. This hydrophobic fusion peptide is then translocated toward the endosomal membrane mediating fusion of the viral envelope with the cell membranes and formation of a fusion pore (Wiley and Skehel, 1987). This conformational change is crucial for the fusogenic activity of HA and for viral entry. Coupled with the fact that the hydrophobic fusion peptide is the only universally conserved epitope in all Influenza viruses, it represents a very attractive target for novel anti-Influenza drugs.

Breast-feeding has been recognised to protect against respiratory and gastrointestinal infections in infants (May, 1988). Milk, besides secretory IgA and IgM, also contains a number of various non-antibody components with known antimicrobial activity, including lactoferrin (May, 1988; Levay and Viljoen, 1995). Lactoferrin (Lf) is an 80-kDa multifunctional cationic glycoprotein belonging to the transferrin family (Levay and Viljoen, 1995). It is present in mucosal secretions, such as tears, saliva, nasal exudate, gastrointestinal fluids, seminal and vaginal fluids, and in granules of polymorphonuclear leukocytes (Levay and Viljoen, 1995). Lf possesses a variety of biological functions such as: influence on iron homeostasis, immunomodulation, and inhibitory activity towards different pathogens (Levay and Viljoen, 1995; Valenti and Antonini, 2005). Bovine lactoferrin (bLf) has been recognized as potent inhibitor of different enveloped viruses such as human cytomegalovirus (Harmsen *et al.,* 1995), Herpes Simplex Viruses types 1 and 2 (Marchetti *et al.,* 1996, 2004, 2009; Ammendolia *et al.,* 2007a), human immunodeficiency virus (HIV) (Harmsen *et al.,* 1995; Swart *et al.,* 1996; Berkhout *et al.,* 2002, 2004), human hepatitis C virus (Ikeda *et al.,* 2000), hantavirus (Murphy *et al.,* 2000), hepatitis B virus (Hara *et al.,* 2002), respiratory syncytial virus (Sano *et al.,* 2003), Sindbis and Semliki Forest viruses (Waarts *et al.,* 2005). BLf, similarly to lactoferrin of other mammalian species, like human lactoferrin, is a glycoprotein folded in two symmetrical lobes (N- and C-lobes), with high sequence homology, possibly resulting from an ancestral gene duplication (Norris *et al.,* 1986; Anderson *et al.,* 1987). Each lobe further consists of two sub-lobes or domains called N1, N2, C1 and C2, respectively. In bovine lactoferrin, the N1 stands for the sequences 1-90 and 251-333, N2 for 91-250, C1 for 345-431 and 593-676, and C2 for 432-592 (Moore *et al.,* 1997; Steijns and van Hooijdonk, 2000). The above domains delimit a $Fe^{3+}$ binding site (Norris *et al.,* 1986; Moore *et al.,* 1997). Although it is generally accepted that, with few exceptions, the inhibiting activity of lactoferrin takes place in the early phases of viral infection, its antiviral effect seems to be exercised in different ways among different viral species. The principal suggested mechanisms for the antiviral activity are a direct binding of lactoferrin to viral particles (Swart *et al.,* 1996; Superti *et al.,* 1997; Marchetti *et al.,* 1999; Pietrantoni *et al.,* 2003; Ammendolia *et al.,* 2007b) or to host cell molecules that the virus uses as a receptor or a co-receptor (Marchetti *et al.,* 1996, 2004; Andersen *et al.,* 2001; Di Biase *et al.,* 2003). An additional effect of bLf on a later intracellular step of virus infection has been described (Superti *et al.,* 1997; Tinari *et al.,* 2005) and, more recently, the inventors of the present invention demonstrated that bLf treatment is able to prevent Influenza virus-induced programmed cell death by interfering with function of caspase 3 (Pietrantoni *et al.,* 2010). This caused the block of nuclear export of viral ribonucleoproteins with consequent viral assembly prevention. In addition, European patent application EP0643074 discloses fragments of the N-lobe of bovine lactoferrin for use in the treatment of influenza virus infection.

In an effort to identify new antiviral therapies effective against Influenza virus, according to the present invention, the inventors have attempted to deeper investigate the mechanism of the anti-Influenza virus effect of bLf and the role of its tryptic fragments (the N- and C-lobes) in the antiviral activity. In particular, they have evaluated the influence of bLf on hemagglutinin-mediated functions. Hemagglutinin has been chosen since it is the major surface protein of the Influenza A virus and is essential to the entry process so representing an attractive target for antiviral therapy. An initial attachment of HA to specific receptors on the host cell surface and a membrane fusion of HA matured by protease digestion are required for virus infection. As a matter of fact, neutralizing compounds targeting HA represent a useful tool in neutralizing viral infection, clearing virus, and suppressing viral spread. Results obtained indicated that lactoferrin is able to bind the $HA_2$ fragment of all Influenza A viruses tested. In particular, the activity of the C-lobe was comparable to that of the entire protein, in the ability to prevent both viral hemagglutination and infection. Importantly, some peptides derived from C- lobe were able to prevent both viral hemagglutination and infection to a much higher potency as compared to the entire protein.

Interestingly, lactoferrin was shown to bind to the $HA_2$ subunit of viral HA, particularly to the fusion peptide, the only universally conserved epitope in all Influenza virus hemagglutinin. This has been demonstrated by N-terminus protein sequencing of subunit samples and by ELISA tests in which the fusion peptide was sufficiently exposed to allow access to bLf. To our knowledge, this is the first demonstration that viral hemagglutination can be inhibited by a specific interaction with the $HA_2$ subunit. As a matter of fact, neutralizing antibodies against Influenza virus have been found to act by two different mechanisms, mirroring the dual functions of hemagglutinin: (i) prevention of attachment to target cells, (ii) inhibition of entry (membrane fusion). The mechanism of neutralization depends on the HA site recognized by the HA-specific molecule. Usually, antibodies against HA act by inhibiting attachment. This occurs as they bind $HA_1$ and physically hinder the interaction with sialic acid receptors on target cells (Edwards and Dimmock, 2001). Some antibodies have been found to prevent membrane fusion; most of them recognize sites in the $HA_2$ region, far away from the receptor-binding site (Ekiert *et al.,* 2009; Sui *et al.,* 2009).

Type A influenza viruses are serologically divided into 16 HA subtypes which are further divided into two major phylogenetic groups: group 1 (subtypes H1, H2, H5, H6, H8, H9, H11, H12, H13, and H16) and group 2 (subtypes H3, H4, H7, H10, H14, and H15). This separation of the 16 subtypes correlates with two distinct basic structures taken by the stalk of the HA (Ekiert *et al.,* 2009). BLf shows activity against viruses expressing hemagglutinin proteins from both group 1 and group 2 as it is able to neutralize isolates from 4 of the 16 influenza A subtypes: H1, H3, H5, and H7. Inventors' working hypothesis is that the interaction of bLf with the highly conserved region of HA (fusion peptide) could allow to a "conformational change" of hemagglutinin so preventing both hemagglutination and infection.

Protein-protein docking calculations, performed to find a putative binding mode of bLf C-lobe to HA, demonstrated the possibility of the bLf targeting a conserved region of HA, close to the fusion peptide. This HA region shares common feature with that of other viral strains, suggesting the possibility to target this part of the hemagglutinin stem for broad spectrum anti-influenza drug design. These calculations provided strong indication about the role of three loops of bLf C-lobe in the interaction with HA, corresponding to the sequences 506-522, 418-429 and 553-563. The two latter sequences are not present in the N-lobe of bLf, which in fact resulted inactive, and the first one presents the 29% of identity with respect to the corresponding portion of the N-lobe. Therefore, three main peptides were identified: SKHSSLDCVLRP (418-429) (SEQ ID NO: 1), AGDDQGLDKCVPNSKEK (506-522) (SEQ ID NO: 2) and NGESTADWAKN (553-563) (SEQ ID No: 3). Because of poor stability of SEQ ID NO:3 peptide, a modified sequence has been designed where the threonine residue is substituted by a homologue serine obtaining a peptide of sequence NGESSADWAKN (SEQ ID NO: 4).

Protein-protein docking calculations suggested the possible role of other loops of bLf C-lobe that can contribute to the binding to HA, corresponding to the sequences 441-454, 478-501, 619-630, 633-637 and 642-659. These loops, like the above mentioned peptides, are bLf solvent exposed regions, located on the C-lobe surface properly to interact with the HA stem region, with a sequence similarity lower than 33% with respect to N-lobe. These peptide are: KANEGLTWNSLKDK (441-454) (SEQ ID NO: 8), TGSCAFDEFFSQSCAPGADPKSR (478-501) (SEQ ID NO: 9), GKNGKNCPDKFC (619-630) (SEQ ID NO: 10), KSETKN (633-637) (SEQ ID NO: 11), NDNTECLAKLGGRPTYEE (642-659) (SEQ ID NO: 12).

The above peptides with SEQ ID NO: 1, 2 and 4 were synthesized and tested for their ability to inhibit hemagglutination and infection.

The results showed that lactoferrin-derived peptides bind to HA and neutralize hemagglutination and *in vitro* infection of different strains of Influenza virus with a very high potency, suggesting that these bLf regions were indeed involved in the bLf inhibition of Influenza virus. Importantly, the peptides are active on viral subtypes belonging to the two major phylogenetic groups of Influenza virus. These peptides may represent a valuable tool for the study of influenza viruses and their receptor-binding interactions, as well as for therapeutic possibilities, that will be examined in *in vivo* tests. The short sequence is an added advantage for both metabolic stability and commercialisation purpose as it can greatly reduce the cost of production.

Therefore, it is a specific object of the present invention a peptide of lactoferrin C-lobe (i.e. a fragment of lactoferrin C-lobe), wherein said peptide is chosen from the group consisting of SKHSSLDCVLRP (418-429) (SEQ ID NO: 1), AGDDQGLDKCVPNSKEK (506-522) (SEQ ID NO: 2), NGESSADWAKN (SEQ ID NO: 4), NGESTADWAKN (553-563) (SEQ ID No: 3), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL. The lactoferrin C-lobe can be a bovine, zebu, wild yak, cow buffalo, goat or sheep lactoferrin C-lobe.

The peptide having sequence SEQ ID NO:1 is present in bovine, wild yak, zebu or cow buffalo lactoferrin C-lobe, SEQ ID NO:2 is a peptide of bovine, zebu, cow buffalo or goat lactoferrin C-lobe, SEQ ID NO: 4 is a peptide of goat or sheep lactoferrin C-lobe or a modified sequence of bovine lactoferrin C-lobe peptide NGESTADWAKN (SEQ ID No: 3) wherein the threonine residue is substituted by a homologue serine. The peptide having sequence SEQ ID NO:3 is present in bovine, wild yak, zebu or cow buffalo lactoferrin C-lobe.

Wild yak, zebu, cow buffalo, goat and sheep lactoferrin C-lobes have identity percentage in comparison to bovine lactoferrin C-lobe of 99.1%, 98.6%, 97.4%, 95.7% and, 94.5%, respectively. SEQ ID NO: 1, 2, 3, 4 have been found in the above specified lactoferrins of previously specified species with 100% identity meanwhile they have not been found in other species.

However, the present invention refers to lactoferrin C-lobe sequence or fragments thereof both prepared by synthesis and extracted by natural source.

The present invention concerns also a peptide of lactoferrin C-lobe, wherein said peptide is chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL, for use as a medicament.

Therefore, the present invention concerns a pharmaceutical composition comprising or consisting of the peptide of lactoferrin C-lobe, wherein said peptide is chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL, as active principle, together with one or more excipients and/or adjuvant pharmaceutically acceptable.

It is further object of the present invention a peptide of lactoferrin C-lobe (i.e. a fragment of lactoferrin C-lobe), wherein said peptide comprises or consists of a peptide chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide and/or lactoferrin C-lobe, or pharmaceutical composition as defined above, for use in the treatment of Influenza virus infection, for instance a type A Influenza virus infection.

The lactoferrin C-lobe can be a bovine, wild yak, zebu, cow buffalo, goat or sheep lactoferrin C-lobe.

Type A Influenza virus infection comprises H1, H2, H5, H6, H8, H9, H11, H12, H13, H16, H3, H4, H7, H10, H14, and H15 subtypes.

As mentioned above, the peptide having sequence SEQ ID NO:1 is present in bovine, wild yak, zebu or cow buffalo lactoferrin C-lobe, SEQ ID NO:2 is a peptide of bovine, zebu, cow buffalo or goat lactoferrin C-lobe, SEQ ID NO: 4 is a peptide of goat or sheep lactoferrin C-lobe or a modified sequence of bovine lactoferrin C-lobe peptide NGESTADWAKN (SEQ ID No: 3) wherein the threonine residue is substituted by a homologue serine. The peptide having sequence SEQ ID NO:3 is present in bovine, wild yak, zebu or cow buffalo lactoferrin C-lobe.

According to the present invention, the lactoferrin C-lobe can be a bovine lactoferrin C-lobe consisting of the following sequence SEQ ID NO:5:

YTRVVWCAVGPEEQKKCQQWSQQSGQNVTCATASTTDDCIVLV
LKGEADALNLDGGYIYTAGKCGLVPVLAENRKSSKHSSLDCVLRPTEGY
LAVAVVKKANEGLTWNSLKDKKSCHTAVDRTAGWNIPMGLIVNQTGSCA
FDEFFSQSCAPGADPKSRLCALCAGDDQGLDKCVPNSKEKYYGYTGAF
RCLAEDVGDVAFVKNDTVWENTNGESTADWAKNLNREDFRLLCLDGTR
KPVTEAQSCHLAVAPNHAVVSRSDRAAHVKQVLLHQQALFGKNGKNCP
DKFCLFKSETKNLLFNDNTECLAKLGGRPTYEEYLGTEYVTAIANLKKCS
TSPLLEACAFLTR.

The present invention now will be described, by illustrative and not limitative way, according to preferred embodiments thereof with particular reference to enclosed drawings wherein:

Figure 1 shows ligand-blot assay of bLf binding to Influenza A Virus H1 N1 subunits. Lane 1: SDS-PAGE (Sodium Dodecyl Sulphate - PolyAcrylamide Gel Electrophoresis) of reduced A/Solomon 3/2006 H1 N1 subunits. Lane 2: lactoferrin blot overlay. Lane 3: specificity control (rabbit anti-Lf antibodies + horseradish peroxidase (HRP)-conjugated anti-rabbit antibodies).

Figure 2 shows ligand-blot assay of bLf binding to Influenza A Virus H3N2 subunits. Lane 1: SDS-PAGE of reduced A/Wisconsin (67/05) H3N2 subunits. Lane 2: lactoferrin blot overlay. Lane 3: specificity control (rabbit anti-Lf antibodies + HRP-conjugated anti-rabbit antibodies).

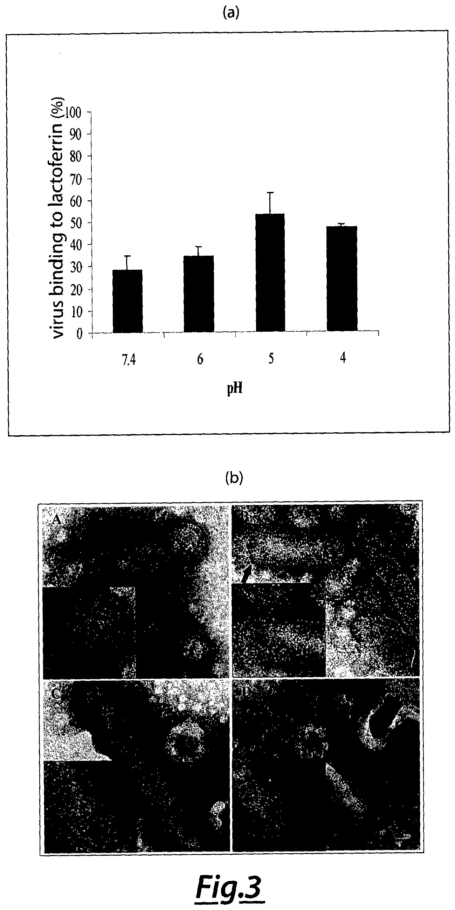

Figure 3 shows (a) influence of pH on Influenza virus binding to bLf by Enzyme-linked immunosorbent assay (ELISA). Influenza virus A/RomaISS/2/08 was treated with different acidic buffers (pH 4.0, 5.0, 6.0, and 7.4) and allowed to bind to plastic-adsorbed bLf. Viral binding was detected by an ELISA by anti-influenza A antibody staining and results are expressed as percentage of virus binding to bLf. Data represents the means of at least three independent experiments. (b) Transmission Electron Microscopy of Influenza virus A/RomaISS/2/08 H1 N1. (A) Influenza virus incubated at neutral pH. Note the spikes are well-ordered. (B) Virus incubated at pH 6.0. Spikes on the virions are still well defined and distinct. (C) Virus incubated at pH 5.0. Note that the spikes on the virions have become disorganized and are no longer well defined or distinct. (D) Virus incubated at pH 4.0. The spikes appear very disordered and discontinue.

Figure 4 shows putative interactions of bLf C-lobe (white ribbon) with HA stem (solid surface light grey): (A) in the cleft between two monomers; (B) close to the fusion peptide (dark grey). Main selected bLf sequences (SEQ ID NO: 1, 2 and 3) correspond to the black loops.

Figure 5 shows sequence alignment of the N- (SEQ ID NO: 7) and C-lobe (SEQ ID NO:5) of bLf. The sequences in bold type correspond to loops supposed to be involved in binding. In bold italic main sequences are highlighted.

Figure 6 shows ligand-blot assay of bLf binding to Influenza SW X-179A swine pandemic (H1N1) subunits. Lane 1: SDS-PAGE of reduced SW X-179A swine pandemic (H1N1) subunits. Lane 2: lactoferrin blot overlay. Lane 3: specificity control (rabbit anti-Lf antibodies + HRP-conjugated anti-rabbit antibodies).

Figure 7 shows N-terminal amino acid sequences determined by Edman degradation method.

**Example 1:** *Study on the effectiveness of the peptides of the present invention against Influenza virus infection.*

Materials and Methods

*Virus strains*

The following Influenza A virus strains were used: A/Solomon 3/2006 H1N1, A/Wisconsin/67/2005 H3N2, and SW X-179A swine pandemic H1 N1 (inactivated vaccines and corresponding subunit preparations); A/Puerto Rico/8/34 H1 N1 (PR8 virus), A/RomaISS/2/08 H1 N1 oseltamivir-sensitive virus, A/Parma/24/09 H1 N1 oseltamivir-resistant virus, A/Parma/05/06 H3N2, Avian 29/05/06 H5N1 (inactivated subunit vaccine), and A/Turkey/Italy/2676/99 H7N1 (kindly provided by Dr. Isabella Donatelli, ISS, Italy). PR8 virus was grown in the allantoic cavities of 10-day-old embryonated chicken eggs. After 48 h at 37 °C, the allantoic fluid was harvested and centrifuged at 5000 rpm for 30 min to remove cellular debris, and virus titers were determined by a hemagglutinin titration and plaque assay according to the standard procedures (Gaush and Smith, 1968; *Kurokawa et al.* 1990). A/RomaISS/2/08 H1N1, A/Parma/24/09 H1N1, and A/Parma/05/06 H3N2 viruses were grown in Madin-Darby canine kidney (MDCK) cells. MDCK cells were grown at 37°C in a humidified atmosphere with 5% $CO_2$ in Minimal Essential Medium (MEM, Gibco; Paisley, UK) containing 1.2 g/L $NaHCO_3$, and supplemented with 10% inactivated fetal calf serum (FCS, Flow Laboratories, Irvine, UK.), 2 mM glutamine, 2% non essential amino acids (Gibco; Paisley, UK), penicillin (100 IU/ml), and streptomycin (100 $\mu$g/ml). Viruses were inoculated onto confluent cell monolayers grown in roller bottles at a multiplicity of infection (m.o.i.) of 1 plaque forming units (p.f.u.)/cell. After 90 min at 35°C, the inoculum was removed and the monolayers were washed three times with Phosphate Buffered Saline (PBS, pH 7.4) and then incubated at 35°C in culture medium containing 2% non essential amino acids (Gibco; Paisley, UK), 4% Bovine Serum Albumin (BSA fraction V, Gibco; Paisley, UK) and 0.5$\mu$g of N-tosyl-L-phenylalanine chloromethyl ketone-treated trypsin (TPCK trypsin; Sigma Chemical Co.; St. Louis, MO, USA). When extensive cytopathic effect (c.p.e.) was observed, infected cultures were frozen and thawed three times, centrifuged (3000 rpm, 10 min), and supernatants were stored at-80°C. The infectivity titer was determined by hemagglutinin titration and plaque assay, according to the standard procedures (Gaush and Smith, 1968; Kurokawa *et al.,* 1990).

*Virus purification*

For virus purification infected allantoic fluid or supernatants from infected cultures were harvested and centrifuged at 1000g for 30 min to remove debris. The virus was pelleted at 25000g for 20 h. The pellet was resuspended in 10 mM HEPES buffer saline (HBS) for 1 h at 4 °C, homogenized, incubated at 20 °C for 45 min, and centrifuged at 2000g for 15 min to remove aggregated virus. The supernatant was placed atop a 0/20/40/60% sucrose density step gradient and centrifuged at 85000g for 2 h. The purified virus was collected from the 20/40% sucrose interface, assayed for protein, quickly frozen, and stored at -80 °C.

*Lactoferrin*

Lactoferrin from bovine milk (bLf), purchased from Morinaga Milk Industries (Zama City, Japan), was deprived of endotoxin as previously described (Pietrantoni *et al.,* 2006). Detoxified bLf was dissolved as stock solution (400 mM) in pyrogen-free PBS. BLf purity was checked by SDS-PAGE (Sodium Dodecyl Sulphate - PolyAcrylamide Gel Electrophoresis) stained with silver nitrate and was judged to be greater than 95%. Protein concentration was determined by UV spectroscopy on the basis of the extinction coefficient of 15.1 (280 nm, 1% solution) (Groves, 1960). The iron saturation rate of bLf, determined by atomic absorption spectrometry, was approximately 19.4%.

*Enzymatic hydrolysis of bLf and HPLC separation, purification and characterisation of N- and C-lobes*

BLf (4 mg/ml) was dissolved in 50 mM ammonium bicarbonate, pH 8.5, and trypsin (from bovine pancreas, TPCK treated, Sigma Chemical Company, St.Louis, MO, USA) digestion was performed at 37°C overnight using an enzyme to substrate ratio of 1:50 w/w. The N- and C-lobes obtained by enzymatic hydrolysis were purified by reverse phase HPLC on a Vydac C18 column (250x10 mm, 5 $\mu$m) using a Waters HPLC System (Datasystem Millenium, HPLC pumps Waters 510, Detector Waters 486). Eluents were 0.1 % trifluoroacetic acid (solvent A) and 0.07% trifluoroacetic acid in 95% acetonitrile (solvent B). The elution was performed by means of a short linear gradient from 35% to 55% solvent B over 20 min. at a flow rate of 3.5 ml/min. and monitored at 220 nm. The trypsin-containing fraction was discharged since it did not co-eluted with fractions containing bLf lobes. In addition, control experiments did not show residual tryptic activity in the digested fractions of bLf since the enzyme underwent auto-hydrolysis and inactivation due to denaturing conditions of HPLC. Electrophoretic analysis (SDS-PAGE) of the HPLC fractions has been carried out using 12.5% gels stained by Comassie Blue R250. Purification and characterisation of bLf N- and C-lobes were carried out as previously described (Superti *et al.,* 2001). Fractions containing bLf lobes were collected, dried in a Speed-Vac centrifuge (Savant), lyophilised twice and stored at -20 °C.

*Peptide synthesis*

Peptide synthesis was performed in Solid Phase Peptide Synthesis (SPPS) with the Fmoc-chemistry by Primm Biotech, Inc., Milan, Italy. The peptide chains have been assembled on a solid support starting from the C-terminus and progressing towards the N-terminus, by repeating three basic steps: deprotection of the Fmoc (9-fluorenylmethyloxycarbonyl) alpha amino group of the amino acid, activation of the next protected amino acid, as an active ester, and coupling. After the desired sequence of amino acids was obtained, the peptide was removed from the polymeric support via TFA (Trifluoroacetic Acid) cleavage with concomitant removal of the protecting groups on the amino acid side-chains.

*Cytotoxicity assay*

To establish the maximal non-cytotoxic dose of bLf and its derivatives, two-fold serial dilutions of each substance in culture medium were incubated at 37°C with confluent MDCK cells grown in 96-well tissue culture microplates (Nalge Europe Ltd, Neerijse, Belgium). After 24 h, the following parameters were evaluated: cell morphology and viability (determined by neutral red staining) were examined by light microscopy and cell proliferation was evaluated quantitatively by microscopic counts after dispersion into individual cells with trypsin. Substance dilutions that did not affect any of these parameters were considered as non-cytotoxic concentrations and utilised for antiviral assays.

*Inhibition of hemagglutination*

Hemagglutination assays were carried out in U-bottomed microtitre plates using 50 $\mu$l of 0.5% suspensions of turkey red blood cells (rbcs) in PBS added to 50 $\mu$l virus serially diluted in PBS. For testing bLf or peptidic fragments for inhibition of hemagglutination, virus in PBS was incubated for 60 min. at 4°C with equal volume of serial dilutions of bLf or its peptidic fragments in PBS, starting from 12.5 $\mu$M. An equal volume of 0.5% turkey rbcs in PBS was then added and allowed to agglutinate. Titres were expressed as the reciprocal of the bLf or its peptidic fragment dilution giving 50% inhibition of hemagglutination by four agglutinating units of virus.

*Neutralization assay*

Neutralization of virus binding to MDCK cells was carried out by incubating serial twofold bLf or peptidic fragment dilutions, starting from 12.5 $\mu$M, in culture medium with equal volumes (0.25 ml) of virus suspension containing $1.10^6$ p.f.u./0.25 ml. In negative controls, culture medium was used instead of bLf or peptidic fragments in the same volume. The mixtures were incubated for 60 min. at 4°C. MDCK cell monolayers, grown in 96 well tissue culture microplates (Nalge Europe Ltd, Neerijse, Belgium) for 24 h at 37°C in 5% $CO_2$-air, were infected with 100 $\mu$l/well (in quadruplicate) of the virus-protein mixtures. It was noted that the virus/cell ratio was very important in this type of neutralization assay and the optimal ratio was to infect 20,000 cells with $2.10^5$ p.f.u. of virus in 96-well plates (m.o.i. 10). After 1 hour adsorption at 37°C, cells were rinsed thoroughly and incubated at 37°C for 24 hours. The viral cytopathic effect (c.p.e.) was measured by neutral red staining. After staining and washes, the healthy MDCK cells took the neutral red dye but not the damaged cells after being infected by Influenza virus. The proportions of healthy cells have been determined by reading the absorbance of neutral red at A540 of each well. The fraction of viable cells in the presence of neutralizing substances was quantified by the staining of cells, where the intensity of the staining was directly proportional to the number of viable cells. Percent neutralization was determined by the formula below:

% neutralization:

$$\frac{A_{540} \text{ of substance testing wells} - A_{540} \text{ of viral control wells}}{A_{540} \text{ of cell control wells} - A_{540} \text{ of viral control wells}}$$

x 100%

The neutralizing substance titer in neutral red assay has been reported as $IC_{50}$, the reciprocal substance dilution at which 50% of MDCK cells were protected from the virus induced killing.

*Neutral red uptake assay*

For neutral red uptake assay, treated and untreated infected cells were stained for 3 h with neutral red (50 $\mu$g/ml, 200 $\mu$l/well, 37°C, 5% $CO_2$); then the cells were washed with Hank's salt solution and fixed for 10 min. at room temperature with 4% formaldehyde, 10% $CaCl_2$ (200 $\mu$l/well). The uptaken dye was extracted for 15 min. at room temperature by 1% acetic acid in 50% ethanol (200 $\mu$l/well) and the damage of the cells by the virus or the possible protection by the compounds were measured at 540 nm in an ELISA-reader.

*Far-western-blot*

Purified viral subunits preparations of Solomon (H1 N1) and Wisconsin (H3N2) strains were resolved by SDS-PAGE, as described by Laemmli (1970) under denaturing or/and non-denaturing conditions on 15% acrylamide gel. Proteins were then transferred from gel to nitrocellulose membranes (Bio-Rad, Hercules, California, USA), using a Semidry Transfer Cell apparatus (Trans-blot, Bio-Rad, Hercules, California, USA) according to Manufacturer's protocol. After transfer, the membranes were blocked with 5% skim-milk solution for 1 h, followed by washing with PBS/0.05% Tween-20 (washing solution). Membranes were then incubated for 90 min. with washing solution containing 1 mg/ml (12.5 $\mu$M) bLf. After extensive washing, membranes were incubated with rabbit anti-lactoferrin antibodies (Sigma Chemical Company, St. Louis, Missouri, USA) in a washing solution for 1 h, washed again, and then incubated 1 h with goat horseradish peroxidase (HRP)-labelled anti-rabbit IgG antibodies (Bio-Rad, Hercules, California, USA). Following extensive washing, staining was achieved by using 3,3',5,5'-tetramethylbenzidine (TMB) substrate kit for peroxidase (Vector Laboratories, Inc., Burlingame, California, USA) according to Manufacturer's instructions. All incubations were carried out at room temperature. Non-specific anti-Lf antibody binding to viral proteins was verified by incubating nitrocellulose strips with rabbit anti-Lf antibodies followed by HRP-conjugated anti-rabbit antibodies.

*Sequencing*

For identification of HA region recognized from bLf in far-western blot, A/Solomon 3/2006 H1 N1 subunits were separated on 15% acrylamide gel in denaturing conditions. Separated proteins were then transferred from gel to polyvinylidene difluoride (PVDF) membrane (Bio-Rad, Hercules, California, USA), using a Semidry Transfer Cell apparatus (Trans-blot, Bio-Rad, Hercules, California, USA) according to Manufacturer's protocol. Transfer was performed using CAPS [3-Cyclohexylamino-1-propanesulfonic acid] 10 mM buffer (pH 11.0) containing 10% methanol. Membrane was then stained by Comassie-Blue R-250 solution (0.25% w/v) in 50% methanol for about 5 min., washed with water and destained in 50% methanol with constant shaking. The protein band of interest was excised, extensively washed with sterile Milli-Q water, and the N-terminal amino acid sequences were determined by Edman degradation method by Primm Biotech, Inc., Milan, Italy.

*Enzyme-linked immunosorbent assay (ELISA)*

ELISA was performed to determine lactoferrin binding to viral particles treated at appropriate pH. The A/RomaISS/2/08 and A/Parma/24/09 viruses were treated with TNE buffer (0.1 M Tris, 1 M NaC1, 0.05 M Na EDTA) at different pH (pH 7.4, 6.0, 5.0, and 4.0). After incubation at 37°C for 15 min., the reaction was neutralized with NaOH and viruses used for binding assay. Flat-bottomed 96-well plates (Nalge Europe Ltd., Neerijse, Belgium) were coated with 0.1 mg/well of lactoferrin in 0.05 M carbonate buffer (pH 9.6) at 4 ° C overnight. The plates were blocked with 10% bovine serum albumin (BSA) in PBS for 2 h at 37 ° C. After washing with PBS containing 0.01% Tween-20 (PBS-T), the plates were incubated at 37 °C for 1 h with 50 $\mu$l of purified viral particles pretreated for 15 min at 37°C with PBS at different pH (pH 7.4, 6.0, 5.0, and 4.0). The plates were washed and incubated at 37 ° C for 1 h with chicken anti-influenza A antibodies (Abcam plc, Cambridge, UK) in PBS containing 1% BSA, washed again and incubated at 37 ° C for 1 h with HRP-conjugated polyclonal rabbit anti-chicken IgG (Sigma Chemical Co.; St. Louis, Missouri, USA). After washing with PBS-T, the peroxidase substrate o-phenylendiamine (OPD) dihydrochloride was added. The developing of colour reaction was stopped by adding 50$\mu$L of 3.0 N HCl. The optical density was read on a multilabel plate reader (PerkinElmer Italia, Monza) at 490 nm.

*Transmission Electron Microscopy (TEM)*

For TEM visualization, purified viral particles were treated for 15 min at 37°C with PBS at different pH (pH 7.4, 6.0, 5.0, and 4.0). After pH neutralization, 20$\mu$l of each sample were absorbed onto carbon-formvar-coated, 400-mesh copper grids, stained with 2% phosphotungstic acid (PTA) (pH 7.0) for 30 s, and observed on a Philips 208S electron microscope at 80 kV.

*Computational methods*

The HA peptide sequences were obtained for the virus strains A/Solomon 3/2006 H1N1, A/Wisconsin/67/2005 H3N2, A/Puerto Rico/8/34 H1 N1 (PR8 virus), A/RomaISS/2/08 H1 N1 and A/Parma/24/09 H1 N1. The above sequences were aligned to those of the HA whose X-ray structures were available in the Protein Data Bank. The BlastX program was used to translate the nucleotide sequence in aminoacid sequence. FASTA sequences alignment was carried out applying the following parameters: algorithm: EMBOSS, method: needle, matrix: Blosum62, gap open penalty: -10, gap extend penalty: -0.5. The same procedure was applied to align the N- and C-lobe of bLf obtained by cutting the complete sequence at the residue number 342.

The protein-protein docking calculations were performed using the X-ray structures of HA with PDB code 2WRG and of the bLf C-lobe of with PDB code 3IBO. The two crystal structures were submitted to the Protein Preparation routine in Maestro (Schrodinger, New York, USA) that allows fixing up receptor structure, eliminating water and sugar molecules, fixing bond order, adding hydrogen atoms, ionising lysine, arginine, glutamate and aspartate residues. To optimize the hydrogen bond network, histidine tautomers and ionization states were predicted, 180° rotations of the terminal X angle of Asn, Gin, and His residues were assigned, and hydroxyl and thiol hydrogen atoms were sampled. For each structure, a brief relaxation was performed using an all-atom constrained minimization carried out with the Impact Refinement module (Impact, Schrodinger, New York, USA) using the OPLS-2005 force field, to reduce steric clashes that may exist in the original PDB structures. The minimization was terminated when the energy converged or the RMSD reached a maximum cutoff of 0.30 Å. The 3IB0 protein was preliminarily passed through the Prime (Prime, Schrodinger, New York, USA) homology modelling routine to complete the structure with missing residues.

So prepared structures were submitted to different docking procedures using four softwares (Autodock-MacroModel, ZDock-RDock, PatchDock-FireDock and ClusPro). All of them comprise a preliminary docking calculation followed by a refinement step.

1. A rigid docking calculation was performed with Autodock, setting the grid on the stem region of just one HA monomer. Docking calculations were carried out employing the Lamarckian Genetic Algorithm as research algorithm implemented in Autodock. The following settings were used: number of grid points in xyz 126 126 126, spacing 1.0 Å, number of the individuals in population 300, maximum number of energy evaluations 2500000, maximum number of generations 30000, hybrid GA-LS, runs 250. After ordering all the conformations by docked energy, the docking poses were clustered by means of ADTools, applying an RMSD cutoff of 0.5 Å. Most representative complexes of each cluster were subsequently fully minimized (with no constraints) with MacroModel (MacroModel, Schrodinger, New York, USA) applying the following settings: force field OPLS-2005, solvent Water, maximum number of iterations: 10000, minimization method PRCG, convergence on gradient with a threshold of 0.05.

2. ZDock and RDock are implemented in Discovery Studio (Discovery Studio, Accelrys, San Diego, USA). Docking calculations were performed setting the following parameters: residues of the upper part of HA and residues of C-lobe of bLf in close contact with the N-lobe were blocked (not explored), 54000 initial docked poses were generated, and the best 2000 poses were ranked by ZRank and then clustered to obtain 100 clusters (RMSD > 10 Å). Most representative complexes of each cluster were submitted to following refinement with RDock applying the default parameters.

3. Patch Dock is a web server (http://bioinfo3d.cs.tau.ac.il/PatchDock) that performs docking calculation using an algorithm based on shape complementarity principles (rigid-body). Default parameters were applied and 100 complexes (clustered with an RMSD cutoff of 4.0 Å) were passed to subsequent refinement with FireDock. This latter is a web server that performs large scale flexible refinement, by restricted interface side-chain rearrangement and by soft rigid-body optimization.

4. ClusPro is a web server (http://cluspro.bu.edu/home.php) that exploits PIPER, an FFT-based docking algorithm, to generate 1000 complexes, that were subsequently clustered. Best cluster representatives were submitted to refinement via Monte Carlo simulation and applying the Semi-Defined programming based Underestimation (SDU) to score the complexes.

All complexes resulting from the four docking calculations were collected and analysed in order to remove unsuitable poses.

The selected complexes were clustered in order to find a consensus between poses generated with different approaches. The clustering was carried out using R (R, The R foundation for statistical computing) and applying the Ward clustering metric.

161 resulting complexes were aligned to each other and visually inspected using PyMOL v 1.2 (PyMOL, DeLano Scientific, San Francisco, USA).

Results

*Interaction of bLf with viral hemagglutinin*

The first step of Influenza virus entry into susceptible cells depends on the interaction between the viral HA and a specific sialic acid-containing cell receptor. As receptor binding of functional HA can be measured by agglutination of turkey erythrocytes, direct interaction between virus and bLf was tested by checking the inhibition of viral hemagglutination activity. As shown in Table 1 (Interaction of bLf with viral HA), concentrations of bLf ranging from about 0.05 pM to 6 nM were able to prevent hemagglutination activity of all tested virus strains. Neither bLf-induced agglutination nor visual evidence of erythrocyte lysis was observed in the above assays, thus bLf inhibition of viral hemagglutination was considered genuine.

Table 1

| Viral strain | subtype | HI titre |
|---|---|---|
| A/Roma-ISS/2/08 (Brisbane-like) (oseltamivir-sensitive) | H1N1 | 6 nM |
| A/Parma/24/09 (Brisbane-like) (oseltamivir-resistant) | H1N1 | 6 nM |
| A/P R/8/34 | H1N1 | 3 nM |
| A/Solomon (3/06) inactivated subunits, vaccine | H1N1 | 5 pM |
| A/Parma/5/06 (Wisconsin-like) | H3N2 | 3 nM |
| A/Wisconsin (67/05) inactivated subunits, vaccine | H3N2 | 0.3 pM |
| SW X-179A 1089/09 inactivated subunits, vaccine | H1N1 | 46.5 fM |
| Avian 29/05/06 inactivated vaccine | H5N1 | 2.9 pM |
| A/Turkey/Italy/2676/99 | H7N1 | 93.1 fM |

*Neutralization of Influenza virus infection by lactoferrin*

It has been examined whether, and to what extent, bLf could affect virus replication. This was tested by neutral red staining measuring of virus-infected MDCK cells as a measure of cell viability. Two strains of the Influenza A/H1 N1 virus subtype (A/Roma-ISS/2/08 and A/Parma/24/09), and one strain of A/H3N2 virus subtype (A/Parma/05/06) were used in these experiments. Virus replication was markedly inhibited by bLf, at concentrations several logs lower than those causing non-specific MDCK cytotoxicity. In order to determine the selectivity index (SI) of lactoferrin, the ratio between the 50% drug cytotoxicity concentration ($CC_{50}$) and the concentration required to inhibit the viral cytopathic effect by 50% ($EC_{50}$) was calculated. SI values were between about $10^6$ and $10^5$ for Influenza A/H1 N1 and A/H3N2 virus subtypes, respectively (data not shown).

*Far-western-blot and protein identification*

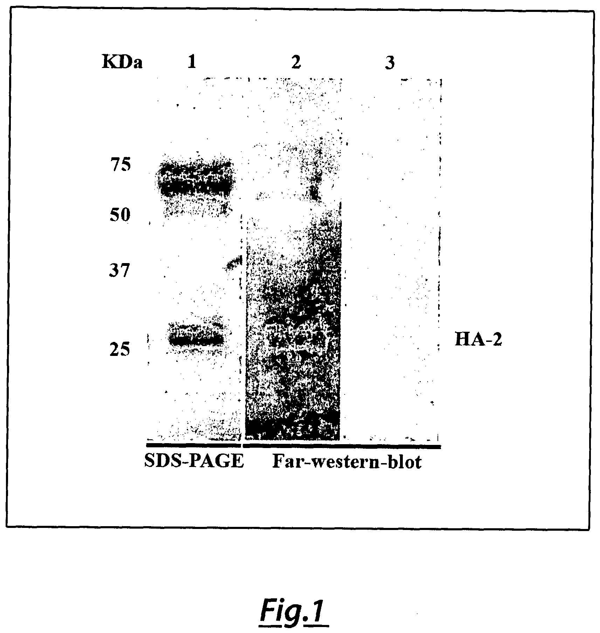

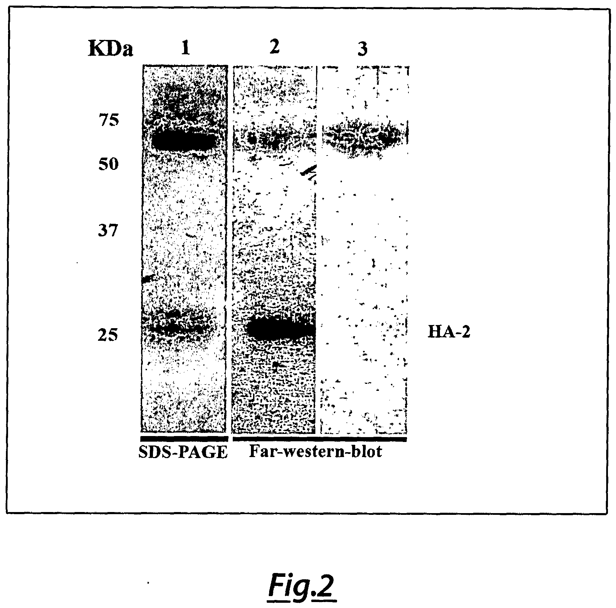

Based on the data reported above, we attempted to identify the actual bLf-virus binding components, and started by examining bLf interaction with virus envelope proteins. To this aim, HA and NA surface antigens present in Solomon (H1N1) and Wisconsin (H3N2) subunit vaccines were separated by electrophoresis and probed with bLf by a far-western blot assay. Figures 1 and 2 show that in both viral strains bLf more consistently binds to a viral protein of apparent molecular mass of 28kDa, tentatively corresponding to the $HA_2$ subunit of HA protein.

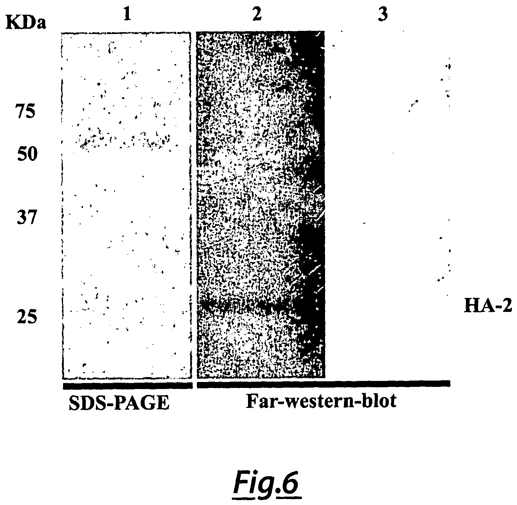

Similar results have been obtained with subunits vaccine of SW X-179A swine pandemic (H1 N1) strain (Figure 6).

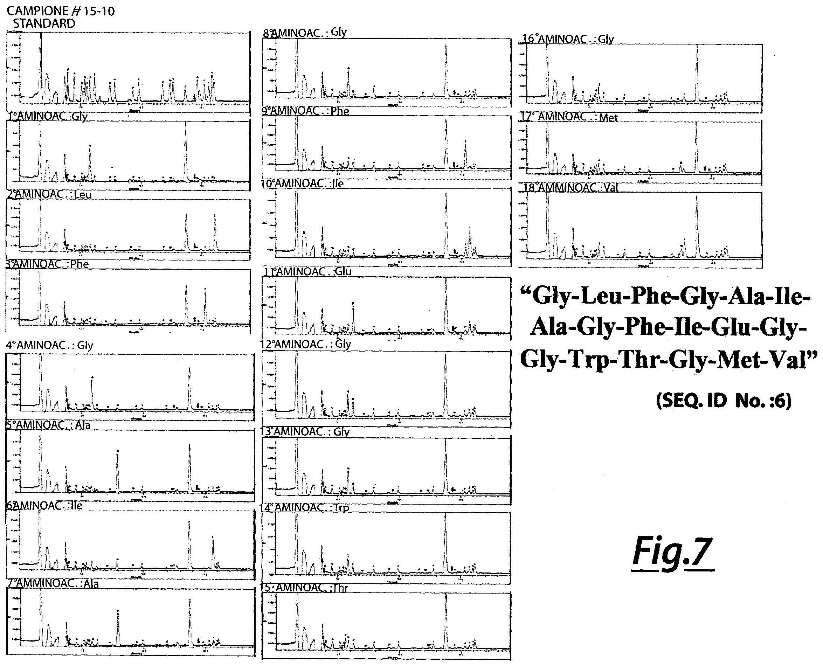

N-terminus protein sequencing of H1 N1 Solomon subunit sample confirmed that the virus component bound by bLf was indeed the $HA_2$ subunit of HA. In particular, the first 18 aminoacids of N-terminus sequence were GLFGAIAGFIEGGWTGMV (SEQ ID No: 6) corresponding to the highly conserved fusion peptide of $HA_2$ subunit (Ekiert *et al.,* 2009) (Figure 7).

*pH assay*

Since bLf recognizes $HA_2$ subunit, which is involved in pH-induced conformational changes during Influenza virus entry into the cells of the susceptible host, experiments were carried out to determine lactoferrin binding to viral particles exposed to different pH values. These experiments were performed, with similar result, with two viral strains, but the data in Figure 3 refer to the Influenza A/RomaISS/2/08 viral strain. BLf was found to bind to the virus with the highest affinity at acidic pH (52.9 +/-9.7% and 34.1 +/-4.1% at pH 5.0 and 6.0, respectively). BLf binding strength was still high at pH 4.0 (47.2 +/-1.4%) (Figure 3a). When Influenza virus was observed by transmission electron microscopy after negative staining with phosphotungstic acid, untreated and acid-treated virus particles markedly differed in their morphology (Figure 3b).

As shown in Figure 3b, at neutral pH, the HA spikes were well-ordered, rectangular structures projecting from the virus membrane (A). Viral particles treated at pH 6.0 showed spikes on the virions still distinct and well defined (B).

After incubation at pH 5.0, the well-ordered appearance was lost, and individual spikes were difficult to discern (C). This pattern was more evident after pH 4.0 treatment, that induced HA spikes to appear very disordered and barely distinguishable (D).

The loss of well-defined spike structure observed at low pH reflects the irreversible conformational change in HA (Ruigrok *et al.,* 1986).

*Effect of N-lobe and C-lobe on viral hemagglutination*

BLf is made of two lobes, named N-lobe and C-lobe, respectively pertaining to the N-terminus and C-terminus moieties of the molecule. We therefore asked which of the two lobes was actually interacting with the $HA_2$ component of the Influenza virus, hence inhibiting viral hemagglutination. Results obtained are reported in Table 2 that shows the activity of N- and C-lobes on viral hemagglutination.

Table 2

| Viral strain | Subtype | N-lobe (aa 1-280) | C-lobe (aa 285-689) | C-lobe (aa 345-689) |
|---|---|---|---|---|
| A/Roma-ISS/2/08 | H1N1 | - | 10 nM | 10 nM |
| A/Parma/24/09 | H1N1 | - | 24 nM | 10 nM |
| A/P R/8/34 | H1N1 | - | 24 nM | 12 nM |
| A/Solomon (3/06) inactivated SU vaccine | H1N1 | - | 5 pM | 5 pM |
| A/Parma/5/06 (Wisconsin-like) | H3N2 | - | 6nM | 6 nM |
| A/Wisconsin (67/05) inactivated SU vaccine | H3N2 | - | 1.6 pM | 1.2 pM |
| SW X-179A 1089/09 inactivated SU vaccine | H1N1 | - | 46.5 fM | 46.5 fM |
| Avian 29/05/06 inactivated vaccine | H5N1 | - | 2.9 pM | 2.4 pM |
| A/Turkey/Italy/2676/99 | H7N1 | - | 93.1 fM | 93.1 fM |

A marked inhibition of viral hemagglutination was obtained with C-lobe whereas N-lobe showed no inhibitory activity.

*Neutralization of Influenza virus infection by N- and C-lobes*

We next examined whether, and to what extent, N- and C lobes of bLf could affect virus replication. Two strains of the Influenza A/H1 N1 virus subtype (A/Roma-ISS/2/08 and A/Parma/24/09), and one strain of A/H3N2 virus subtype (A/Parma/05/06) were used in these experiments. As expected, virus infection was not affected by N-lobe treatment whereas replication was highly inhibited by C-lobe. SI values were 2.5-5 fold higher than bLf (data not shown).

*Protein-protein docking*

The data above show that bLf interacts with HA and suggest a possible interaction between the C-lobe of bLf and the fusion peptide ($HA_2$ subunit) of HA. In order to gain more insight into the binding mode between bLf and HA a protein-protein docking protocol has been setup because of the lack of structural data (X-ray or NMR) about the interaction between these two proteins.

For the docking calculations the X-ray structure of the C-lobe of bLf with PDB code 3IB0 was used, as this portion is responsible for the bLf activity (see above). The crystal structure of HA with PDB code 2WRG was selected because it showed the highest sequence similarity with the HAs isolated from the viral strain of H1 N1 used in the assays. Table 3 shows the percentage of sequence identity and similarity of indicated strains hemagglutinins with the crystal structure 2WRG.

Table 3

| Viral strain | subype | % Identity[a] | % Similarity[a] |
|---|---|---|---|
| A/Solomon (3/06) inactivated subunits, vaccine | H1N1 | 82 | 88 |
| A/Roma-ISS/2/08 | H1N1 | 84 | 90 |
| A/Parma/24/09 | H1N1 | 84 | 89 |
| A/PR/8/34 | H1N1 | 86 | 90 |
| [a] values calculated with the Multiple Sequence Viewer of Maestro. | | | |

Noteworthy, major differences were found in the residues of the $HA_1$ subunit, whereas the $HA_2$ sequence was perfectly conserved among all of the considered HAs belonging to the H1 N1 strains, demonstrating that this region is an interesting target for anti-Influenza therapies.

Docking calculations were performed using four different software (ZDOCK, PatchDock, AutoDock, ClusPro) selected as they use different approaches. Results obtained from all procedures were analysed with the aim to find a consensus: finding common binding modes applying diverse docking protocols can improve the reliability of the observed results; it is well known that protein-protein docking is a challenging computational approach that provide many results because of the large dimensions of the interacting protein surfaces.

All the simulations were carried out using the C-lobe of bLf to speed up calculation, as experimental data showed that N-lobe was inactive. Moreover, as the Far-Western-blot analysis demonstrated the binding at the $HA_2$ subunit, binding poses involving this region of HA were selected for following analysis.

The results were analysed to eliminate all the unsuitable binding poses, involving not accessible regions of bLf or HA.

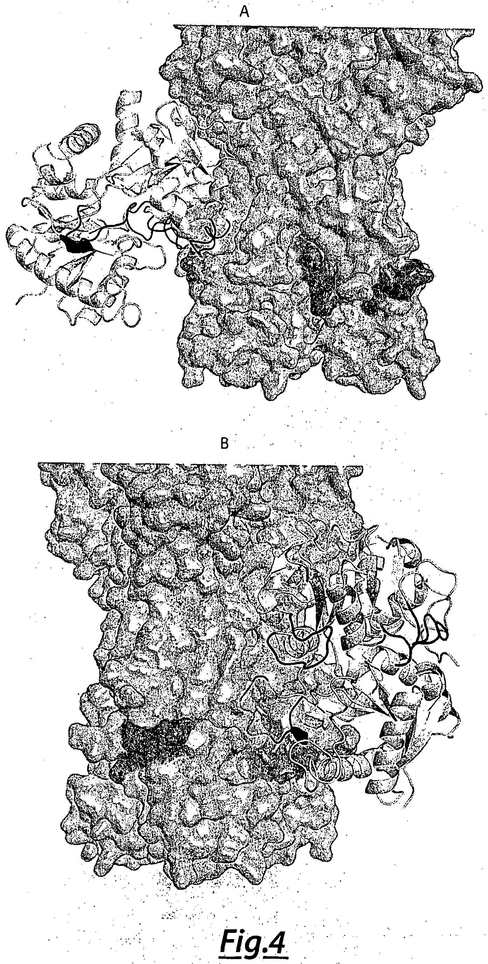

As shown in Figure 4, most of the binding poses concentrated on two regions of the HA stem: the first one in the cleft between two monomers (Figure 4A) and the second one very close to the fusion peptide (Figure 4B). The latter binding site seems of particular interest as it corresponds to the region targeted from universal antibodies. Therefore binding to this region could explain why bLf inhibition is exerted toward all the viral strains under analysis.

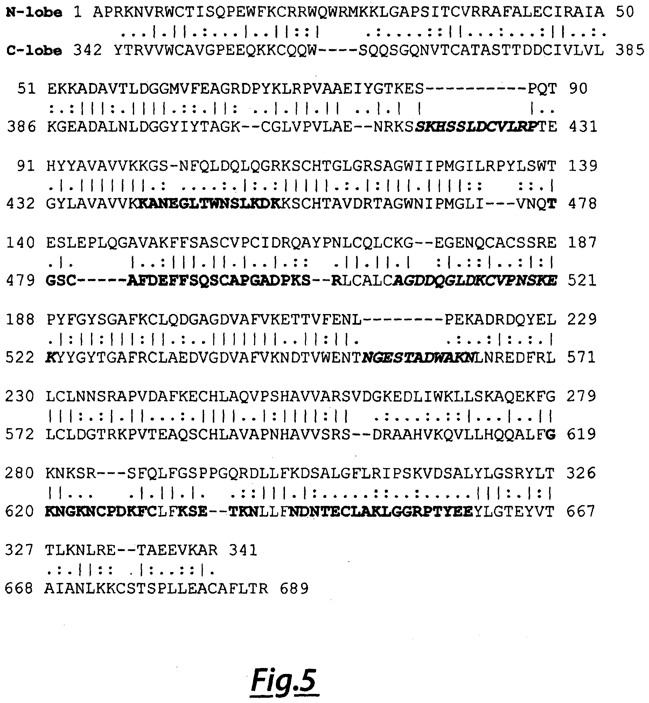

C-lobe of bLf can bind in many different ways to HA: interaction to hemagglutinin is most frequently mediated by three loops: SKHSSLDCVLRP (aa 418-429)(SEQ ID NO:1), NGESTADWAKN, (aa 553-563) (SEQ ID NO:3) and AGDDQGLDKCVPNSKEK (aa 506-522) (SEQ ID NO:2). However also other sequences corresponding to solvent exposed, accessible loops of bLf can contribute to the overall interaction with HA: KANEGLTWNSLKDK (441-454) (SEQ ID NO: 8), TGSCAFDEFFSQSCAPGADPKSR (478-501) (SEQ ID NO: 9), GKNGKNCPDKFC (619-630) (SEQ ID NO: 10), KSETKN (633-637) (SEQ ID NO: 11), NDNTECLAKLGGRPTYEE (642-659) (SEQ ID NO: 12) Alignment of the sequences of the C- and the inactive N-lobe (Figure 5) demonstrates that sequences 418-429 and 553-563 correspond to loops that are not present in the N-lobe, while the other selected loops show a sequence identity with respect to N-lobe < 33% (see Table 4).

Because of poor stability of NGESTADWAKN peptide, a modified sequence has been designed where the threonine residue is substituted by a homologue serine, i.e. NGESSADWAKN (SEQ ID NO: 4). The peptides with SEQ ID NO: 1, 2 and 4 were tested in HI and Nt assays.

Table 4

| SEQ ID NO | Residues | Sequence | Identity vs N-lobe (%) |
|---|---|---|---|
| 1 | 418-429 | SKHSSLDCVLRP (SEQ ID NO:1) | 8 |
| 2 | 506-522 | AGDDQGLDKCVPNSKEK (SEQ ID NO:2) | 29 |
| 3 | 553-563 | NGESTADWAKN (SEQ ID NO:3) | 0 |
| 4 | - | NGESSADWAKN (SEQ ID NO:4) | - |
| 8 | 441-454 | KANEGLTWNSLKDK (SEQ ID NO:8) | 14 |
| 9 | 478-501 | TGSCAFDEFFSQSCAPGADPKSR (SEQ ID NO:9) | 33 |
| 10 | 619-630 | GKNGKNCPDKFC (SEQ ID NO:10) | 33 |
| 11 | 633-637 | KSETKN (SEQ ID NO:11) | 12 |
| 12 | 642-659 | NDNTECLAKLGGRPTYEE (SEQ ID NO:12) | 6 |

*Interaction of bLf derived peptides with viral hemagglutinin*

The direct interaction between virus and bLf-derived peptides was tested by checking the inhibition of viral hemagglutination. As shown in Table 4, SKHSSLDCVLRP (amino acid residues 418-429) (SEQ ID NO:1, AGDDQGLDKCVPNSKEK (amino acid residues 506-522) (SEQ ID NO:2), and NGESSADWAKN (SEQ ID NO: 4) were able to prevent hemagglutinating activity of all tested virus strains at higher extent compared to the entire protein.

Table 5

| Viral strain | subtype | SEQ ID No:1 HI titre | SEQ ID NO:2 HI titre | SEQ ID No:4 HI titre |
|---|---|---|---|---|
| A/Roma-ISS/2/08 | H1N1 | 1.4 pM | 1.4 pM | 0.7 fM |
| A/Parma/24/09 | H1N1 | 1.4 pM | 1.4 pM | 0.3 fM |
| A/PR/8/34 | H1N1 | 0.7 nM | 0.35 nM | 0.7 fM |
| A/Solomon (3/06) inactivated subunits, vaccine | H1N1 | 46.5 fM | 46.5 fM | 93 fM |
| A/Parma/5/06 (Wisconsin-like) | H3N2 | 0.7 pM | 0.7 pM | 0.3 pM |
| A/Wisconsin (67/05) inactivated subunits, vaccine | H3N2 | 23.2 fM | 11.6 fM | 5.8 fM |
| SW X-179A 1089/09 inactivated SU vaccine | H1N1 | 5.8 fM | 46 fM | 0.3 fM |
| Avian 29/05/06 inactivated vaccine | H5N1 | 18 fM | 70 fM | 93.1 fM |
| A/Turkey/Italy/2676/99 | H7N1 | 18 fM | 46.5 fM | 5.8 fM |

As observed for bLf, neither peptide-induced agglutination nor visual evidence of erythrocyte lysis was detected in the above assays, thus also peptide inhibition of viral hemagglutination was considered genuine.

*Neutralization of Influenza virus infection in MDCK cells by bLf-derived peptides*

We examined whether, and to what extent, bLf-derived peptides were able to affect virus replication. Two strains of the Influenza A/H1 N1 virus subtype (A/Roma-ISS/2/08 and A/Parma/24/09), and one strain of A/H3N2 virus subtype (A/Parma/05/06) were used in these experiments. As shown in Tables 6, 7, and 8, bLf-derived peptides were better inhibitors than the entire protein, their selectivity index being about one or two order of magnitude higher, depending on virus strain. Concerning NGESSADWAKN (SEQ ID NO:4), the SI in H1 N1 (oseltamivir resistant strain) infected cells reaches values 500 times higher than bLf.

Table 6

| *In vitro* antiviral activity of SKHSSLDCVLRP (SEQ ID NO:1) towards Influenza virus infection | | | | |
|---|---|---|---|---|
| Viral strain | subype | $CC_{50}$ * | $EC_{50}$ ° | SI^ |
| A/Roma-ISS/2/08 | H1N1 | > 25 $\mu$M | 4 pM | >6.25.10[6] |
| A/Parma/24/09 | H1N1 | > 25 $\mu$M | 3.1 pM | >8.10[6] |
| A/Parma/05/06 | H3N2 | > 25 $\mu$M | 5.8 pM | >4.3.10[6] |

Table 7

| *In vitro* antiviral activity of AGDDQGLDKCVPNSKEK (SEQ ID NO:2) towards Influenza virus infection | | | | |
|---|---|---|---|---|
| Viral strain | subype | $CC_{50}$ * | $EC_{50}$ ° | SI^ |
| A/Roma-ISS/2/08 | H1N1 | > 25 $\mu$M | 3.7 pM | >6.75.10[6] |
| A/Parma/24/09 | H1N1 | > 25 $\mu$M | 3.4 pM | >7.35.10[6] |

(continued)

| *In vitro* antiviral activity of AGDDQGLDKCVPNSKEK (SEQ ID NO:2) towards Influenza virus infection | | | | |
|---|---|---|---|---|
| Viral strain | subtype | $CC_{50}$ * | $EC_{50}$ ° | SI^ |
| A/Parma/05/06 | H3N2 | > 25 μM | 7.3 pM | >3.42.$10^6$ |

Table 8

| *In vitro* antiviral activity of NGESSADWAKN (SEQ ID NO:4) towards Influenza virus infection | | | | |
|---|---|---|---|---|
| Viral strain | subtype | $CC_{50}$ * | $EC_{50}$ ° | SI^ |
| A/Roma-ISS/2/08 | H1N1 | > 25 μM | 225 fM | >1.11.$10^8$ |
| A/Parma/24/09 | H1N1 | > 25 μM | 50 fM | >5.$10^8$ |
| A/Parma/05/06 | H3N2 | > 25 μM | 22.5 pM | >1.11.$10^6$ |
| *$CC_{50}$ cytotoxic concentration 50%, °$EC_{50}$ effective concentration 50%, ^SI (selectivity index)=$CC_{50}$/$EC_{50}$ | | | | |

References

Ammendolia MG, Marchetti M, Superti F. (2007a) Bovine lactoferrin prevents the entry and intercellular spread of herpes simplex virus type 1 in Green Monkey Kidney cells. Antiviral Res. 76:252-262.

Ammendolia MG, Pietrantoni A, Tinari A, Valenti P, Superti F. 2007b. Bovine lactoferrin inhibits echovirus endocytic pathway by interacting with viral structural polypeptides. Antiviral Res. 73:151-60.

Andersen JH, Osbakk SA, Vorland LH, Traavik T, and Gutteberg TJ. 2001. Lactoferrin and cyclic lactoferricin inhibit the entry of human cytomegalovirus into human fibroblasts. Antiviral Res. 51:141-149.

Anderson BF, Baker HM, Dodson EJ, Norris GE, Rumball SV, Waters JM, Baker EN. 1987. Structure of human lactoferrin at 3.2-A resolution. Proc. Natl. Acad. Sci. USA 84:1769-1773.

Berkhout B, van Wamel JL, Beljaars L, Meijer DK, Visser S, Floris R. 2002. Characterization of the anti-HIV effects of native lactoferrin and other milk proteins and protein-derived peptides. Antiviral Res. 55:341-355.

Berkhout B, Floris R, Recio I, Visser S. 2004. The antiviral activity of the milk protein lactoferrin against the human immunodeficiency virus type 1. Biometals 17:291-294.

Cheng PK, To AP, Leung TW, Leung PC, Lee CW, Lim WW. 2010. Oseltamivir- and amantadine-resistant influenza virus A (H1N1), Emerg. Infect. Dis. 16:155-156.

Dharan NJ, Gubareva LV, Meyer JJ, Okomo-Adhiambo M, McClinton RC, Marshall SA, St George K, Epperson S, Brammer L, Klimov AI, Bresee JS, Fry AM, Oseltamivir-Resistance Working Group. 2009. Infections with oseltamivir-resistant influenza A(H1N1) virus in the United States. JAMA 30:1034-1041.

Di Biase AM, Pietrantoni A, Tinari A, Siciliano R, Valenti P, Antonimi G, Seganti L, Superti F. 2003. Heparin-interacting sites of bovine lactoferrin are involved in anti-adenovirus activity. J. Med. Virol. 69:495-502.

Dolin R, Reichman RC, Madore HP, Maynard R, Linton PN, Webber-Jones J. 1982. A controlled trial of amantadine and rimantadine in the prophylaxis of influenza A infection. N. Engl. J. Med. 307:580-584.

Edwards M, Dimmock NJ. 2001. Hemagglutinin 1-specific immunoglobulin G and Fab molecules mediate postattachment neutralization of influenza A virus by inhibition of an early fusion event J. Virol. 75:10208- 10218.

Ekiert DC, Bhabha G, Elsliger MA, Friesen RH, Jongeneelen M, Throsby M, Goudsmit J, Wilson IA. 2009. Antibody recognition of a highly conserved influenza virus epitope. Science 324:246-251

Fiore AE, Shay DK, Broder K, Iskander JK, Uyeki TM, Mootrey G, Bresee JS, Cox NS. Centers for Disease Control and Prevention (CDC). Advisory Committee on Immunization Practices (ACIP) 2008. Prevention and control of influenza: recommendations of the Advisory Committee on Immunization Practices (ACIP). MMWR Recomm. Rep. 57(RR-7):1-60.

Gaush CR, Smith TF. 1968. Replication and plaque assay of influenza virus in an established line of canine kidney cells. Appl. Microbiol. 16:588-594.

Groves ML 1960. The isolation of a red protein from milk. J. Am. Chem. Soc. 82:3345-3350.

Hara K, Ikeda M, Saito S, Matsumoto S, Numata K, Kato N, Tanaka K, Sekihara H. 2002. Lactoferrin inhibits hepatitis B virus infection in cultured human hepatocytes. Hepatol. Res. 24:228-235.

Harmsen MC, Swart PJ, de B'ethune MP, Pawels R, De Clercq E, Th'e TH, Meijer DKF. 1995. Antiviral effects of plasma and milk proteins: lactoferrin shows a potent activity against both human immunodeficiencyvirus and human cytomegalovirus replication in vitro. J. Infect. Dis. 172:380-388

Hayden FG 2006. Antiviral resistance in influenza viruses - implications for management and pandemic response. N. Engl. J. Med. 354:785-788.

Kiso M, Mitamura K, Sakai-Tagawa Y, Shiraishi K, Kawakami C, Kimura K, Hayden FG, Sugaya N, Kawaoka Y. 2004. Resistant influenza A viruses in children treated with oseltamivir: descriptive study. Lancet 364:759-765.

Kurokawa M, Ochiai H, Nakajima K, Niwayama S. 1990. Inhibitory effect of protein kinase C inhibitor on the replication of influenza type A virus. J. Gen. Virol. 71:2149-2155.

Ikeda M, Nozaki A, Sugiyama K, Tanaka T, Naganuma A, Tanaka K, Sekihara H, Shimotohno K, Saito M, Kato N. 2000. Characterization of antiviral activity of lactoferrin against hepatitis C virus infection in human cultured cells. Virus Res. 66:51-63.

Ilyushina NA, Bovin NV, Webster RG, Govorkova, EA. 2006. Combination chemotherapy, a potential strategy for reducing the emergence of drug-resistant influenza A variants. Antiviral Res. 70: 121-131.

Laemmli UK 1970. Cleavage of structural prroteins during the assembly of the head of bacteriophage T4. Nature 227:680-685.

Levay PF, Viljoen M. 1995. Lactoferrin: a general review. Haematologica 80:252-267.

Marchetti, Longhi C, Conte MP, Pisani S, Valenti P, Seganti L. 1996. Lactoferrin inhibits herpes simplex virus type 1 adsorption to Vero cells, Antiviral Res. 29:221-231.

Marchetti M, Superti F, Ammendolia MG, Rossi P, Valenti P, Seganti L. 1999. Inhibition of poliovirus type 1 infection by iron-, manganeseand zinc-saturated lactoferrin. Med. Microbiol. Immunol. 187:199-204.

Marchetti M, Trybala E, Superti F, Johansson M, Bergstrom T. 2004. Inhibition of herpes simplex virus infection by lactoferrin is dependent on interference with the virus binding to glycosaminoglycans. Virology 18:405-413.

Marchetti M, Ammendolia MG, Superti F 2009. Glycosaminoglycans are not indispensable for the anti-herpes simplex virus type 2 activity of lactoferrin. Biochimie 91:155-159.

May JT. 1988. Microbial contaminants and antimicrobial properties of human milk. Microbiol. Sci. 5:42-46.

Murphy ME, Kariwa H, Mizutani T, Yoshimatsu K, Arikawa J, Takashima I. 2000. In vitro antiviral activity of lactoferrin and ribavirin upon hantavirus. Arch. Virol. 145:1571-1582.

Norris GE, Gartner AL, Anderson BF, Ward J, Baker EN, Rumball SV, Baker HM. 1986. Preliminary crystallographic studies on bovine lactoferrin. J. Mol. Biol. 191:143-145.

Pietrantoni A, Di Biase AM, Tinari A, Marchetti M, Valenti P, Seganti L, Superti F. 2003. Bovine lactoferrin inhibits adenovirus infection by interacting with viral structural polypeptides. Antimicrob. Agents Chemother. 47:2688-91.

Pietrantoni A, Ammendolia MG, Tinari A, Siciliano R, Valenti P, Superti F. 2006. Bovine lactoferrin peptidic fragments involved in inhibition of Echovirus 6 in vitro infection. Antiviral Res 69:98-106.

Pietrantoni A, Dofrelli E, Tinari A, Ammendolia MG, Puzelli S, Fabiani C, Donatelli I, Superti F. 2010. Bovine lactoferrin inhibits influenza A virus induced programmed cell death in vitro. Biometals 23:465-475.

Ruigrok RW, Wrigley NG, Calder LJ, Cusack S, Wharton SA, Brown EB, Skehel JJ. 1986. Electron microscopy of the low pH structure of influenza virus haemagglutinin. EMBO J. 5:41-49.

Sano H, Nagai K, Tsutsumi H, Kuroki K. 2003. Lactoferrin and surfactant protein A exhibit distinct binding specificity to F protein and differently modulate respiratory syncytial virus infection. Eur. J. Immunol. 33:2894-2902.

Skehel JJ, Wiley DC. 2000. Receptor binding and membrane fusion in virus entry: the influenza hemagglutinin. Annu. Rev. Biochem. 69:531-569.

Smith JR, Rayner CR, Donner B, Wollenhaupt M, Klumpp K, Dutkowski R. 2011 Oseltamivir in seasonal, pandemic, and avian influenza: a comprehensive review of 10-years clinical experience. Adv Ther. 28:927-959.

Steijns JM, van Hooijdonk AC. 2000. Occurrence, structure, biochemical properties and technological characteristics of lactoferrin. Br. J. Nutr.84 Suppl 1:S11-17.

Sui J, Hwang WC, Perez S, Wei G, Aird D, Chen LM, Santelli E, Stec B, Cadwell G, Ali M, Wan H, Murakami A, Yammanuru A, Han T, Cox NJ, Bankston LA, Donis RO, Liddington RC, Marasco WA. 2009. Structural and functional bases for broad-spectrum neutralization of avian and human influenza A viruses. Nat. Struct. Mol. Biol. 16:265-273.

Superti F, Ammendolia MG, Valenti P, Seganti L. 1997. Antirotaviral activity of milk proteins: lactoferrin prevents rotavirus infection in the enterocyte-like cell line HT-29. Med. Microbiol. Immunol. 186:83-91.

Superti F, Siciliano R, Rega B, Giansanti F, Valenti P, Antonini G, 2001. Involvement of bovine lactoferrin metal saturation, sialic acid and protein fragments in the inhibition of rotavirus infection. Biochim. Biophys. Acta 1528:107-115.

Swart PJ, Kuipers ME, Smit C, Pauwels R, de B'ethune MP, DeClercq E, Meijer DKF, Huisman JG. 1996. Antiviral effects of milk proteins: acylation results in polyanionic compounds with potent activity against human immunodeficiency virus types 1 and 2 in vitro. AIDS Res. Hum. Retroviruses 12:769-775.

Tinari A, Pietrantoni A, Ammendolia MG, Valenti, P, Superti F. 2005. Inhibitory activity of bovine lactoferrin against echovirus induced programmed cell death in vitro. Int. J. Antimicrob. Agents 25:433-438.

Valenti P, Antonini G. 2005. Lactoferrin: an important host defence against microbial and viral attack. Cell. Mol. Life Sci. 62:2576-2587.

Waarts BL, Aneke OJ, Smit JM, Kimata K, Bittman R, Meijer DK, Wilschut J. 2005. Antiviral activity of human lactoferrin: inhibition of alphavirus interaction with heparan sulfate. Virology 333:284-292.

Wiley DC, Skehel JJ. 1987 The structure and function of the hemagglutinin membrane glycoprotein of influenza virus. Annu. Rev. Biochem. 56:365-394.

Wilson IA, Skehel JJ, Wiley DC. 1981. Structure of the hemagglutinin membrane glycoprotein of influenza virus at 3Å resolution. Nature 289:366-373

**Claims**

1. Peptide of lactoferrin C-lobe, wherein said peptide is chosen from the group consisting of SKHSSLDCVLRP (SEQ ID NO: 1), AGDDQGLDKCVPNSKEK (SEQ ID NO: 2), NGESSADWAKN (SEQ ID NO: 4), NGESTADWAKN (SEQ ID No: 3), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL.

2. Peptide of lactoferrin C-lobe, wherein said peptide is chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL, for use as a medicament.

3. Pharmaceutical composition comprising or consisting of the peptide of lactoferrin C-lobe, wherein said peptide is chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide; wherein said fragment of said peptide is different from CVLRP (SEQ ID NO: 13), VLRP (SEQ ID NO: 14), CVL, RP, VL, as active principle, together with one or more excipients and/or adjuvant pharmaceutically acceptable.

4. Peptide of lactoferrin C-lobe, wherein said peptide comprises or consists of a peptide chosen from the group consisting of SKHSSLDCVLRP (SEQ ID NO: 1), AGDDQGLDKCVPNSKEK (SEQ ID NO: 2), NGESSADWAKN (SEQ ID NO: 4), or mixture of said peptides or mixture of at least one of said peptide and fragment of said peptide and/or lactoferrin C-lobe, or pharmaceutical composition as defined in claim 3, for use in the treatment of Influenza virus infection.

5. Peptide of lactoferrin C-lobe, or mixture of said peptides or mixture of at least one of said peptide and fragment of said peptide and/or lactoferrin C-lobe, according to claim 4, or pharmaceutical composition as defined in claim 3, for use according to claim 4, wherein said lactoferrin C-lobe is a bovine lactoferrin C-lobe consisting of the following sequence SEQ ID NO:5:

YTRVVWCAVGPEEQKKCQQWSQQSGQNVTCATASTTDDCIVLV
LKGEADALNLDGGYIYTAGKCGLVPVLAENRKSSKHSSLDCVLRPTEGY
LAVAVVKKANEGLTWNSLKDKKSCHTAVDRTAGWNIPMGLIVNQTGSCA
FDEFFSQSCAPGADPKSRLCALCAGDDQGLDKCVPNSKEKYYGYTGAF
RCLAEDVGDVAFVKNDTVWENTNGESTADWAKNLNREDFRLLCLDGTR
KPVTEAQSCHLAVAPNHAVVSRSDRAAHVKQVLLHQQALFGKNGKNCP
DKFCLFKSETKNLLFNDNTECLAKLGGRPTYEEYLGTEYVTAIANLKKCS
TSPLLEACAFLTR

6. Peptide of lactoferrin C-lobe, or mixture of said peptides or mixture of at least one of said peptide and fragment of said peptide and/or lactoferrin C-lobe according to anyone of claims 4-5, or pharmaceutical composition as defined in claim 3, for use according to anyone of the claims 4-5, wherein Influenza virus infection is type A Influenza virus infection.

7. Peptide of lactoferrin C-lobe, or mixture of said peptides or mixture of at least one of said peptide and fragment of said peptide and/or lactoferrin C-lobe according to anyone of claims 4-6, or pharmaceutical composition as defined in claim 3, for use according to anyone of the claims 4-6, wherein type A Influenza virus infection is chosen from the group consisting of H1, H2, H5, H6, H8, H9, H11, H12, H13, H16, H3, H4, H7, H10, H14, and H15 subtypes.

**Patentansprüche**

1. Peptid von Lactoferrin-C-Lappen, wobei das Peptid aus der Gruppe ausgewählt wird bestehend aus SKHSSLDCVLRP (SEQ ID NO: 1), AGDDQGLDKCVPNSKEK (SEQ ID NO: 2), NGESSADWAKN (SEQ ID NO : 4), NGESTADWAKN (SEQ ID No: 3) oder einer Mischung der Peptide oder Mischung von mindestens einem des Peptids und Fragments des Peptids; wobei das Fragment des Peptids von CVLRP (SEQ ID NO: 13), VLRP (SEQ ID NO: 14), CVL, RP, VL verschieden ist.

2. Peptid von Lactoferrin-C-Lappen, wobei das Peptid aus der Gruppe ausgewählt wird bestehend aus SKHSSLDCVLRP (SEQ ID NO: 1), AGDDQGLDKCVPNSKEK (SEQ ID NO: 2), NGESSADWAKN (SEQ ID NO : 4) oder einer Mischung der Peptide oder Mischung von mindestens einem des Peptids und Fragments des Peptids; wobei das Fragment des Peptids von CVLRP (SEQ ID NO: 13), VLRP (SEQ ID NO: 14), CVL, RP, VL verschieden ist, zur Verwendung als Medikament.

3. Pharmazeutische Zusammensetzung umfassend oder bestehend aus dem Peptid von Lactoferrin C-Lappen , wobei das Peptid aus der Gruppe ausgewählt wird bestehend aus SKHSSLDCVLRP (SEQ ID NO: 1), AGDDQGLDKCVPNSKEK (SEQ ID NO: 2), NGESSADWAKN (SEQ ID NO : 4) oder einer Mischung der Peptide oder einer Mischung von mindestens einem des Peptids und Fragments des Peptids; wobei das Fragment des Peptids von CVLRP (SEQ ID NO: 13), VLRP (SEQ ID NO: 14), CVL, RP, VL verschieden ist, als Wirkstoff zusammen mit einem oder mehreren pharmazeutisch akzeptablen Trägern und/oder Hilfsmitteln.

4. Peptid von Lactoferrin-C-Lappen, wobei das Peptid ein Peptid umfasst oder daraus besteht, das aus der Gruppe ausgewählt wird bestehend aus SKHSSLDCVLRP (SEQ ID NO: 1), AGDDQGLDKCVPNSKEK (SEQ ID NO: 2), NGESSADWAKN (SEQ ID NO : 4) oder einer Mischung der Peptide oder einer Mischung von mindestens einem des Peptids und Fragments des Peptids und/oder Lactoferrin-C-Lappen, oder pharmazeutische Zusammensetzung wie in Anspruch 3 definiert, zur Verwendung bei der Behandlung von Grippevirusinfektion.

5. Peptid von Lactoferrin-C-Lappen oder Mischung der Peptide oder Mischung von mindestens einem des Peptids und Fragments des Peptids und/oder Lactoferrin-C-Lappen nach Anspruch 4, oder pharmazeutische Zusammensetzung wie in Anspruch 3 definiert zur Verwendung nach Anspruch 4,
wobei der Lactoferrin-C-Lappen ein Rinder-Lactoferrin-C-Lappen ist, der aus der folgenden Sequenz SEQ ID NO: 5 besteht:

YTRVVWCAVGPEEQKKCQQWSQQSGQNVTCATASTTDDCIVLVLKGEADALNLD

GGYIYTAGKCGLVPVLAENRKSSKHSSLDCVLRPTEGYLAVAVVKKANEGLTWNSL

KDKKSCHTAVDRTAGWNIPMGLIVNQTGSCAFDEFFSQSCAPGADPKSRLCALCA

GDDQGLDKCVPNSKEKYYGYTGAFRCLAEDVGDVAFVKNDTVWENTNGESTAD

WAKNLNREDFRLLCLDGTRKPVTEAQSCHLAVAPNHAVVSRSDRAAHVKQVLLH

QQALFGKNGKNCPDKFCLFKSETKNLLFNDNTECLAKLGGRPTYEEYLGTEYVTAIA

NLKKCSTSPLLEACAFLTR

**6.** Peptid von Lactoferrin-C-Lappen oder Mischung der Peptide oder Mischung von mindestens einem des Peptids und Fragments des Peptids und/oder Lactoferrin-C-Lappen nach einem der Ansprüche 4 - 5 oder pharmazeutisch Zusammensetzung wie in Anspruch 3 definiert zur Verwendung nach einem der Ansprüche 4 - 5, wobei die Grippevirusinfektion eine Infektion von Grippevirus Typ A ist.

**7.** Peptid von Lactoferrin-C-Lappen oder Mischung der Peptide oder Mischung von mindestens einem des Peptids und Fragments des Peptids und/oder Lactoferrin-C-Lappen nach einem der Ansprüche 4 - 6 oder pharmazeutische Zusammensetzung wie in Anspruch 3 definiert, zur Verwendung nach einem der Ansprüche 4 - 6, wobei die Infektion mit Grippevirus von Typ A aus der Gruppe ausgewählt ist bestehend aus H1-, H2-, H5-, H6-, H8-, H9-, H11-, H12-, H13-, H-16-, H3-, H4-, H7-, H10-, H14- and H15-Subtypen.

## Revendications

**1.** Peptide du lobe C de la lactoferrine, dans lequel ledit peptide est choisi dans le groupe constitué des suivants : SKHSSLDCVLRP (SEQ ID NO : 1), AGDDQGLDKCVPNSKEK (SEQ ID NO: 2), NGESSADWAKN (SEQ ID NO: 4), NGESTADWAKN (SEQ ID NO : 3) ou un mélange desdits peptides ou un mélange de l'un dudit peptide et du fragment dudit peptide ; dans lequel ledit fragment dudit peptide est différent des CVLRP (SEQ ID NO : 13), VLRP (SEQ ID NO : 14), CVL, RP, VL.

**2.** Peptide du lobe C de la lactoferrine, dans lequel ledit peptide est choisi dans le groupe constitué des suivants : SKHSSLDCVLRP (SEQ ID NO : 1), AGDDQGLDKCVPNSKEK (SEQ ID NO : 2), NGESSADWAKN (SEQ ID NO : 4), ou un mélange desdits peptides ou un mélange de l'un dudit peptide et du fragment dudit peptide ; dans lequel ledit fragment dudit peptide est différent des CVLRP (SEQ ID NO: 13), VLRP (SEQ ID NO: 14), CVL, RP, VL pour utilisation comme médicament.

**3.** Composition pharmaceutique comprenant ou est constituée du peptide du lobe C de la lactoferrine, dans laquelle ledit peptide est choisi dans le groupe constitué des suivants : SKHSSLDCVLRP (SEQ ID NO : 1), AGDDQGLDKCVPNSKEK (SEQ ID NO : 2), NGESSADWAKN (SEQ ID NO: 4), ou un mélange desdits peptides ou un mélange de l'un dudit peptide et du fragment dudit peptide ; dans lequel ledit fragment dudit peptide est différent des CVLRP (SEQ ID NO : 13), VLRP (SEQ ID NO : 14), CVL, RP, VL, comme principe actif, conjointement avec un ou plusieurs excipients et/ou adjuvants pharmaceutiquement acceptables.

**4.** Peptide du lobe C de la lactoferrine, dans lequel ledit peptide comprend ou est constitué d'un peptide choisi dans le groupe constitué des suivants : SKHSSLDCVLRP (SEQ ID NO: 1), AGDDQGLDKCVPNSKEK (SEQ ID NO: 2), NGESSADWAKN (SEQ ID NO : 4), ou un mélange desdits peptides ou un mélange de l'un dudit peptide et du fragment dudit peptide et/ou du lobe C de la lactoferrine, ou composition pharmaceutique selon la revendication 3, pour utilisation dans le traitement de l'infection par le virus de la grippe.

**5.** Peptide du lobe C de la lactoferrine ou mélange desdits peptides ou mélange d'au moins l'un dudit peptide et du fragment dudit peptide et/ou du lobe C de la lactoferrine, selon la revendication 4 ou composition pharmaceutique selon la revendication 3 pour utilisation selon la revendication 4,
dans lequel ou laquelle le lobe C de la lactoferrine est un lobe C de lactoferrine bovine constitué de la séquence suivante SEQ ID NO : 5 :

YTRVVWCAVGPEEQKKCQQWSQQSGQNVTCATASTTDDCIVLV

LKGEADALNLDGGYIYTAGKCGLVPVLAENRKSSKHSSLDCVLRPTEGY

LAVAVVKKANEGLTWNSLKDKKSCHTAVDRTAGWNIPMGLIVNQTGSCA

FDEFFSQSCAPGADPKSRLCALCAGDDQGLDKCVPNSKEKYYGYTGAF

RCLAEDVGDVAFVKNDTVWENTNGESTADWAKNLNREDFRLLCLDGTR

KPVTEAQSCHLAVAPNHAVVSRSDRAAHVKQVLLHQQALFGKNGKNCP

DKFCLFKSETKNLLFNDNTECLAKLGGRPTYEEYLGTEYVTAIANLKKCSTSPLLEACAFLTR.

**6.** Peptide du lobe C de la lactoferrine ou mélange desdits peptides ou mélange d'au moins l'un dudit peptide et du fragment dudit peptide et/ou du lobe C de la lactoferrine, selon l'une quelconque des revendications 4 à 5 ou composition pharmaceutique selon la revendication 3 pour utilisation selon l'une quelconque des revendications 4 à 5, dans lequel ou laquelle l'infection du virus de la grippe est une infection du virus de la grippe du type A.

**7.** Peptide du lobe C de la lactoferrine ou mélange desdits peptides ou mélange d'au moins l'un dudit peptide et du fragment dudit peptide et/ou du lobe C de la lactoferrine, selon l'une quelconque des revendications 4 à 6 ou composition pharmaceutique selon la revendication 3 pour utilisation selon l'une quelconque des revendications 4 à 6, dans lequel ou laquelle l'infection du virus de la grippe du type A est choisie dans le groupe constitué de sous-types H1, H2, H5, H6, H8, H9, H11, H12, H13, H16, H3, H4, H7, H10, H14 et H15.

EP 2 780 365 B1

*Fig.1*

29

Fig.2

(a)

(b)

*Fig.3*

A

B

*Fig.4*

```
N-lobe   1 APRKNVRWCTISQPEWFKCRRWQWRMKKLGAPSITCVRRAFALECIRAIA  50
             ...|.||.:...|..||::|      .:....::||...:...:||..:.
C-lobe 342 YTRVVWCAVGPEEQKKCQQW----SQQSGQNVTCATASTTDDCIVLVL 385


  51 EKKADAVTLDGGMVFEAGRDPYKLRPVAAEIYGTKES----------PQT  90
       :.:|||:.||||.::.||: ..|.||.|| ..|.| |       |..
 386 KGEADALNLDGGYIYTAGK--CGLVPVLAE--NRKSSKHSSLDCVLRPTE 431


  91 HYYAVAVVKKGS-NFQLDQLQGRKSCHTGLGRSAGWIIPMGILRPYLSWT 139
       .|.|||||||.: .....:.|:.:|||||.:.|:|||.||||::  :: .|
 432 GYLAVAVVKKANEGLTWNSLKDKKSCHTAVDRTAGWNIPMGLI---VNQT 478


 140 ESLEPLQGAVAKFFSASCVPCIDRQAYPNLCQLCKG--EGENQCACSSRE 187
       .|.      |..:|||.||.|..|.:: .||.||.| :|.::|..:|:|
 479 GSC-----AFDEFFSQSCAPGADPKS--RLCALCAGDDQGLDKCVPNSKE 521


 188 PYFGYSGAFKCLQDGAGDVAFVKETTVFENL-------PEKADRDQYEL 229
       .|:||:|||:||.:..|||||||..||:||.     .:..:|:.:.|
 522 KYYGYTGAFRCLAEDVGDVAFVKNDTVWENTNGESTADWAKNLNREDFRL 571


 230 LCLNNSRAPVDAFKECHLAQVPSHAVVARSVDGKEDLIWKLLSKAQEKFG 279
       |||:.:|.||...:.||||..|:||||:|| .:...:.::|...|..||
 572 LCLDGTRKPVTEAQSCHLAVAPNHAVVSRS--DRAAHVKQVLLHQQALFG 619


 280 KNKSR---SFQLFGSPPGQRDLLFKDSALGFLRIPSKVDSALYLGSRYLT 326
       ||...   .|.||.|.  .::|||.|:.....:.::.....|||:.|:|
 620 KNGKNCPDKFCLFKSE--TKNLLFNDNTECLAKLGGRPTYEEYLGTEYVT 667


 327 TLKNLRE--TAEEVKAR 341
       .:.||::  .|:..::|.
 668 AIANLKKCSTSPLLEACAFLTR 689
```

*Fig.5*

**Fig.6**

CAMPIONE # 15-10
STANDARD

**"Gly-Leu-Phe-Gly-Ala-Ile-
Ala-Gly-Phe-Ile-Glu-Gly-
Gly-Trp-Thr-Gly-Met-Val"**

(SEQ. ID No. :6)

*Fig.7*

EP 2 780 365 B1

## REFERENCES CITED IN THE DESCRIPTION

### Patent documents cited in the description

- EP 0643074 A
- IT 267699
- WO RM2011A000606 PCT


### Non-patent literature cited in the description

- **AMMENDOLIA MG ; MARCHETTI M ; SUPERTI F.** Bovine lactoferrin prevents the entry and intercellular spread of herpes simplex virus type 1 in Green Monkey Kidney cells. *Antiviral Res,* 2007, vol. 76, 252-262
- **AMMENDOLIA MG ; PIETRANTONI A ; TINARI A ; VALENTI P ; SUPERTI F.** Bovine lactoferrin inhibits echovirus endocytic pathway by interacting with viral structural polypeptides. *Antiviral Res,* 2007, vol. 73, 151-60
- **ANDERSEN JH ; OSBAKK SA ; VORLAND LH ; TRAAVIK T ; GUTTEBERG TJ.** Lactoferrin and cyclic lactoferricin inhibit the entry of human cytomegalovirus into human fibroblasts. *Antiviral Res,* 2001, vol. 51, 141-149
- **ANDERSON BF ; BAKER HM ; DODSON EJ ; NORRIS GE ; RUMBALL SV ; WATERS JM ; BAKER EN.** Structure of human lactoferrin at 3.2-A resolution. *Proc. Natl. Acad. Sci. USA,* 1987, vol. 84, 1769-1773
- **BERKHOUT B ; VAN WAMEL JL ; BELJAARS L ; MEIJER DK ; VISSER S ; FLORIS R.** Characterization of the anti-HIV effects of native lactoferrin and other milk proteins and protein-derived peptides. *Antiviral Res,* 2002, vol. 55, 341-355
- **BERKHOUT B ; FLORIS R ; RECIO I ; VISSER S.** The antiviral activity of the milk protein lactoferrin against the human immunodeficiency virus type 1. *Biometals,* 2004, vol. 17, 291-294
- **CHENG PK ; TO AP ; LEUNG TW ; LEUNG PC ; LEE CW ; LIM WW.** Oseltamivir- and amantadine-resistant influenza virus A (H1N1). *Emerg. Infect. Dis.,* 2010, vol. 16, 155-156
- **DHARAN NJ ; GUBAREVA LV ; MEYER JJ ; OKOMO-ADHIAMBO M ; MCCLINTON RC ; MARSHALL SA ; ST GEORGE K ; EPPERSON S ; BRAMMER L ; KLIMOV AI.** Infections with oseltamivir-resistant influenza A(H1N1) virus in the United States. *JAMA,* 2009, vol. 30, 1034-1041
- **DI BIASE AM ; PIETRANTONI A ; TINARI A ; SICILIANO R ; VALENTI P ; ANTONIMI G ; SEGANTI L ; SUPERTI F.** Heparin-interacting sites of bovine lactoferrin are involved in anti-adenovirus activity. *J. Med. Virol.,* 2003, vol. 69, 495-502
- **DOLIN R ; REICHMAN RC ; MADORE HP ; MAYNARD R ; LINTON PN ; WEBBER-JONES J.** A controlled trial of amantadine and rimantadine in the prophylaxis of influenza A infection. *N. Engl. J. Med.,* 1982, vol. 307, 580-584
- **EDWARDS M ; DIMMOCK NJ.** Hemagglutinin 1-specific immunoglobulin G and Fab molecules mediate postattachment neutralization of influenza A virus by inhibition of an early fusion event J. *Virol.,* 2001, vol. 75, 10208-10218
- **EKIERT DC ; BHABHA G ; ELSLIGER MA ; FRIESEN RH ; JONGENEELEN M ; THROSBY M ; GOUDSMIT J ; WILSON IA.** Antibody recognition of a highly conserved influenza virus epitope. *Science,* 2009, vol. 324, 246-251
- **FIORE AE ; SHAY DK ; BRODER K ; ISKANDER JK ; UYEKI TM ; MOOTREY G ; BRESEE JS ; COX NS.** Centers for Disease Control and Prevention (CDC). Advisory Committee on Immunization Practices (ACIP) 2008. Prevention and control of influenza: recommendations of the Advisory Committee on Immunization Practices (ACIP). *MMWR Recomm. Rep.,* vol. 57 (RR-7), 1-60
- **GAUSH CR ; SMITH TF.** Replication and plaque assay of influenza virus in an established line of canine kidney cells. *Appl. Microbiol.,* 1968, vol. 16, 588-594
- **GROVES ML.** The isolation of a red protein from milk. *J. Am. Chem. Soc.,* 1960, vol. 82, 3345-3350
- **HARA K ; IKEDA M ; SAITO S ; MATSUMOTO S ; NUMATA K ; KATO N ; TANAKA K ; SEKIHARA H.** Lactoferrin inhibits hepatitis B virus infection in cultured human hepatocytes. *Hepatol. Res.,* 2002, vol. 24, 228-235

- **HARMSEN MC ; SWART PJ ; DE B'ETHUNE MP ; PAWELS R ; DE CLERCQ E ; TH'E TH ; MEIJER DKF.** Antiviral effects of plasma and milk proteins: lactoferrin shows a potent activity against both human immunodeficiencyvirus and human cytomegalovirus replication in vitro. *J. Infect. Dis.,* 1995, vol. 172, 380-388
- **HAYDEN FG.** Antiviral resistance in influenza viruses - implications for management and pandemic response. *N. Engl. J. Med.,* 2006, vol. 354, 785-788
- **KISO M ; MITAMURA K ; SAKAI-TAGAWA Y ; SHIRAISHI K ; KAWAKAMI C, KIMURA K ; HAYDEN FG ; SUGAYA N ; KAWAOKA Y.** Resistant influenza A viruses in children treated with oseltamivir: descriptive study. *Lancet,* 2004, vol. 364, 759-765
- **KUROKAWA M ; OCHIAI H ; NAKAJIMA K ; NIWAYAMA S.** Inhibitory effect of protein kinase C inhibitor on the replication of influenza type A virus. *J. Gen. Virol.,* 1990, vol. 71, 2149-2155
- **IKEDA M ; NOZAKI A ; SUGIYAMA K ; TANAKA T ; NAGANUMA A ; TANAKA K ; SEKIHARA H ; SHIMOTOHNO K ; SAITO M ; KATO N.** Characterization of antiviral activity of lactoferrin against hepatitis C virus infection in human cultured cells. *Virus Res.,* 2000, vol. 66, 51-63
- **ILYUSHINA NA ; BOVIN NV ; WEBSTER RG ; GOVORKOVA, EA.** Combination chemotherapy, a potential strategy for reducing the emergence of drug-resistant influenza A variants. *Antiviral Res.,* 2006, vol. 70, 121-131
- **LAEMMLI UK.** Cleavage of structural prroteins during the assembly of the head of bacteriophage T4. *Nature,* 1970, vol. 227, 680-685
- **LEVAY PF ; VILJOEN M.** Lactoferrin: a general review. *Haematologica,* 1995, vol. 80, 252-267
- **MARCHETTI ; LONGHI C ; CONTE MP ; PISANI S ; VALENTI P ; SEGANTI L.** Lactoferrin inhibits herpes simplex virus type 1 adsorption to Vero cells. *Antiviral Res.,* 1996, vol. 29, 221-231
- **MARCHETTI M ; SUPERTI F ; AMMENDOLIA MG ; ROSSI P ; VALENTI P ; SEGANTI L.** Inhibition of poliovirus type 1 infection by iron-, manganeseand zinc-saturated lactoferrin. *Med. Microbiol. Immunol.,* 1999, vol. 187, 199-204
- **MARCHETTI M ; TRYBALA E ; SUPERTI F ; JOHANSSON M ; BERGSTROM T.** Inhibition of herpes simplex virus infection by lactoferrin is dependent on interference with the virus binding to glycosaminoglycans. *Virology,* 2004, vol. 18, 405-413
- **MARCHETTI M ; AMMENDOLIA MG ; SUPERTI F.** Glycosaminoglycans are not indispensable for the anti-herpes simplex virus type 2 activity of lactoferrin. *Biochimie,* 2009, vol. 91, 155-159
- **MAY JT.** Microbial contaminants and antimicrobial properties of human milk. *Microbiol. Sci.,* 1988, vol. 5, 42-46
- **MURPHY ME ; KARIWA H ; MIZUTANI T ; YOSHIMATSU K ; ARIKAWA J ; TAKASHIMA I.** In vitro antiviral activity of lactoferrin and ribavirin upon hantavirus. *Arch. Virol.,* 2000, vol. 145, 1571-1582
- **NORRIS GE ; GARTNER AL ; ANDERSON BF ; WARD J ; BAKER EN ; RUMBALL SV ; BAKER HM.** Preliminary crystallographic studies on bovine lactoferrin. *J. Mol. Biol.,* 1986, vol. 191, 143-145
- **PIETRANTONI A ; DI BIASE AM ; TINARI A ; MARCHETTI M ; VALENTI P ; SEGANTI L ; SUPERTI F.** Bovine lactoferrin inhibits adenovirus infection by interacting with viral structural polypeptides. *Antimicrob. Agents Chemother.,* 2003, vol. 47, 2688-91
- **PIETRANTONI A ; AMMENDOLIA MG ; TINARI A ; SICILIANO R ; VALENTI P ; SUPERTI F.** Bovine lactoferrin peptidic fragments involved in inhibition of Echovirus 6 in vitro infection. *Antiviral Res,* 2006, vol. 69, 98-106
- **PIETRANTONI A ; DOFRELLI E ; TINARI A ; AMMENDOLIA MG ; PUZELLI S ; FABIANI C ; DONATELLI I ; SUPERTI F.** Bovine lactoferrin inhibits influenza A virus induced programmed cell death in vitro. *Biometals,* 2010, vol. 23, 465-475
- **RUIGROK RW ; WRIGLEY NG ; CALDER LJ ; CUSACK S ; WHARTON SA ; BROWN EB ; SKEHEL JJ.** Electron microscopy of the low pH structure of influenza virus haemagglutinin. *EMBO J.,* 1986, vol. 5, 41-49
- **SANO H ; NAGAI K ; TSUTSUMI H ; KUROKI K.** Lactoferrin and surfactant protein A exhibit distinct binding specificity to F protein and differently modulate respiratory syncytial virus infection. *Eur. J. Immunol.,* 2003, vol. 33, 2894-2902
- **SKEHEL JJ ; WILEY DC.** Receptor binding and membrane fusion in virus entry: the influenza hemagglutinin. *Annu. Rev. Biochem.,* 2000, vol. 69, 531-569
- **SMITH JR ; RAYNER CR ; DONNER B ; WOLLENHAUPT M ; KLUMPP K ; DUTKOWSKI R.** Oseltamivir in seasonal, pandemic, and avian influenza: a comprehensive review of 10-years clinical experience. *Adv Ther.,* 2011, vol. 28, 927-959
- **STEIJNS JM ; VAN HOOIJDONK AC.** Occurrence, structure, biochemical properties and technological characteristics of lactoferrin. *Br. J. Nutr.,* 2000, vol. 84 (1), 11-17
- **SUI J ; HWANG WC ; PEREZ S ; WEI G ; AIRD D ; CHEN LM ; SANTELLI E ; STEC B ; CADWELL G ; ALI M.** Structural and functional bases for broad-spectrum neutralization of avian and human influenza A viruses. *Nat. Struct. Mol. Biol.,* 2009, vol. 16, 265-273

- **SUPERTI F ; AMMENDOLIA MG ; VALENTI P ; SEGANTI L.** Antirotaviral activity of milk proteins: lactoferrin prevents rotavirus infection in the enterocyte-like cell line HT-29. *Med. Microbiol. Immunol.,* 1997, vol. 186, 83-91
- **SUPERTI F ; SICILIANO R ; REGA B ; GIANSANTI F ; VALENTI P ; ANTONINI G.** Involvement of bovine lactoferrin metal saturation, sialic acid and protein fragments in the inhibition of rotavirus infection. *Biochim. Biophys. Acta,* 2001, vol. 1528, 107-115
- **SWART PJ ; KUIPERS ME ; SMIT C ; PAUWELS R ; DE B'ETHUNE MP ; DECLERCQ E ; MEIJER DKF ; HUISMAN JG.** Antiviral effects of milk proteins: acylation results in polyanionic compounds with potent activity against human immunodeficiency virus types 1 and 2 in vitro. AIDS Res. Hum. *Retroviruses,* 1996, vol. 12, 769-775
- **TINARI A ; PIETRANTONI A ; AMMENDOLIA MG ; VALENTI, P ; SUPERTI F.** Inhibitory activity of bovine lactoferrin against echovirus induced programmed cell death in vitro. *Int. J. Antimicrob. Agents,* 2005, vol. 25, 433-438
- **VALENTI P ; ANTONINI G.** Lactoferrin: an important host defence against microbial and viral attack. *Cell. Mol. Life Sci.,* 2005, vol. 62, 2576-2587
- **WAARTS BL ; ANEKE OJ ; SMIT JM ; KIMATA K ; BITTMAN R ; MEIJER DK ; WILSCHUT J.** Antiviral activity of human lactoferrin: inhibition of alphavirus interaction with heparan sulfate. *Virology,* 2005, vol. 333, 284-292
- **WILEY DC ; SKEHEL JJ.** The structure and function of the hemagglutinin membrane glycoprotein of influenza virus. *Annu. Rev. Biochem.,* 1987, vol. 56, 365-394
- **WILSON IA ; SKEHEL JJ ; WILEY DC.** Structure of the hemagglutinin membrane glycoprotein of influenza virus at 3Å resolution. *Nature,* 1981, vol. 289, 366-373

SEQUENCE LISTING

<110> Istituto Superiore di sanità Universita degli Studi G. D'Annunzio chieti - Pescara

<120> Lactoferrin derived peptides for use as broad-spectrum inhibitors of Influenza virus infection

<130> PCT28866

<150> RM2011A000606
<151> 2011-11-16

<160> 15

<170> PatentIn version 3.5

<210> 1
<211> 12
<212> PRT
<213> Artificial sequence

<220>
<223> bovine lactoferrin peptide from 418 aa to 429 aa

<400> 1

```
Ser Lys His Ser Ser Leu Asp Cys Val Leu Arg Pro
1               5                   10
```

<210> 2
<211> 17
<212> PRT
<213> Artificial Sequence

<220>
<223> bovine lactoferrin peptide from 506 aa to 522 aa

<400> 2

```
Ala Gly Asp Asp Gln Gly Leu Asp Lys Cys Val Pro Asn Ser Lys Glu
1               5                   10                  15

Lys
```

<210> 3

<211> 11
<212> PRT
<213> Artificial Sequence

<220>
<223> bovine lactoferrin peptide from 553 aa to 563 aa

<400> 3

```
                    Asn Gly Glu Ser Thr Ala Asp Trp Ala Lys Asn
                    1               5                   10
```

<210> 4
<211> 11
<212> PRT
<213> Artificial Sequence

<220>
<223> modified SEQ ID NO:3 wherein the threonine residue is substituted by a homologue serine

<400> 4

```
                    Asn Gly Glu Ser Ser Ala Asp Trp Ala Lys Asn
                    1               5                   10
```

<210> 5
<211> 348
<212> PRT
<213> Artificial sequence

<220>
<223> bovine lactoferrin C-lobe peptide

<400> 5

```
Tyr Thr Arg Val Val Trp Cys Ala Val Gly Pro Glu Glu Gln Lys Lys
1               5               10                  15

Cys Gln Gln Trp Ser Gln Gln Ser Gly Gln Asn Val Thr Cys Ala Thr
            20              25                  30

Ala Ser Thr Thr Asp Asp Cys Ile Val Leu Val Leu Lys Gly Glu Ala
        35              40                  45

Asp Ala Leu Asn Leu Asp Gly Gly Tyr Ile Tyr Thr Ala Gly Lys Cys
    50              55                  60

Gly Leu Val Pro Val Leu Ala Glu Asn Arg Lys Ser Ser Lys His Ser
65              70                  75                  80

Ser Leu Asp Cys Val Leu Arg Pro Thr Glu Gly Tyr Leu Ala Val Ala
            85              90                  95

Val Val Lys Lys Ala Asn Glu Gly Leu Thr Trp Asn Ser Leu Lys Asp
            100             105             110

Lys Lys Ser Cys His Thr Ala Val Asp Arg Thr Ala Gly Trp Asn Ile
        115             120             125

Pro Met Gly Leu Ile Val Asn Gln Thr Gly Ser Cys Ala Phe Asp Glu
    130             135             140

Phe Phe Ser Gln Ser Cys Ala Pro Gly Ala Asp Pro Lys Ser Arg Leu
145             150             155             160

Cys Ala Leu Cys Ala Gly Asp Asp Gln Gly Leu Asp Lys Cys Val Pro
            165             170             175

Asn Ser Lys Glu Lys Tyr Tyr Gly Tyr Thr Gly Ala Phe Arg Cys Leu
            180             185             190
```

```
Ala Glu Asp Val Gly Asp Val Ala Phe Val Lys Asn Asp Thr Val Trp
        195             200             205

Glu Asn Thr Asn Gly Glu Ser Thr Ala Asp Trp Ala Lys Asn Leu Asn
        210             215             220

Arg Glu Asp Phe Arg Leu Leu Cys Leu Asp Gly Thr Arg Lys Pro Val
225             230             235             240

Thr Glu Ala Gln Ser Cys His Leu Ala Val Ala Pro Asn His Ala Val
            245             250             255

Val Ser Arg Ser Asp Arg Ala Ala His Val Lys Gln Val Leu Leu His
            260             265             270

Gln Gln Ala Leu Phe Gly Lys Asn Gly Lys Asn Cys Pro Asp Lys Phe
        275             280             285

Cys Leu Phe Lys Ser Glu Thr Lys Asn Leu Leu Phe Asn Asp Asn Thr
    290             295             300

Glu Cys Leu Ala Lys Leu Gly Gly Arg Pro Thr Tyr Glu Glu Tyr Leu
305             310             315             320

Gly Thr Glu Tyr Val Thr Ala Ile Ala Asn Leu Lys Lys Cys Ser Thr
            325             330             335

Ser Pro Leu Leu Glu Ala Cys Ala Phe Leu Thr Arg
        340             345
```

<210> 6
<211> 18
<212> PRT
<213> Artificial sequence

<220>
<223> first 18 aminoacids of N-terminus sequence corresponding to the highly conserved fusion peptide of viral hemagglutinin (HA) HA2 subunit of H1N1 Solomon

<400> 6

```
Gly Leu Phe Gly Ala Ile Ala Gly Phe Ile Glu Gly Gly Trp Thr Gly
1               5               10              15

Met Val
```

<210> 7
<211> 341
<212> PRT
<213> Artificial sequence

<220>
<223> Bovine lactoferrin N-lobe peptide

<400> 7

```
Ala Pro Arg Lys Asn Val Arg Trp Cys Thr Ile Ser Gln Pro Glu Trp
1               5               10              15

Phe Lys Cys Arg Arg Trp Gln Trp Arg Met Lys Lys Leu Gly Ala Pro
        20              25              30

Ser Ile Thr Cys Val Arg Arg Ala Phe Ala Leu Glu Cys Ile Arg Ala
        35              40              45

Ile Ala Glu Lys Lys Ala Asp Ala Val Thr Leu Asp Gly Gly Met Val
    50              55              60

Phe Glu Ala Gly Arg Asp Pro Tyr Lys Leu Arg Pro Val Ala Ala Glu
65              70              75              80

Ile Tyr Gly Thr Lys Glu Ser Pro Gln Thr His Tyr Tyr Ala Val Ala
            85              90              95

Val Val Lys Lys Gly Ser Asn Phe Gln Leu Asp Gln Leu Gln Gly Arg
        100             105             110

Lys Ser Cys His Thr Gly Leu Gly Arg Ser Ala Gly Trp Ile Ile Pro
        115             120             125

Met Gly Ile Leu Arg Pro Tyr Leu Ser Trp Thr Glu Ser Leu Glu Pro
    130             135             140

Leu Gln Gly Ala Val Ala Lys Phe Phe Ser Ala Ser Cys Val Pro Cys
145             150             155             160

Ile Asp Arg Gln Ala Tyr Pro Asn Leu Cys Gln Leu Cys Lys Gly Glu
            165             170             175

Gly Glu Asn Gln Cys Ala Cys Ser Ser Arg Glu Pro Tyr Phe Gly Tyr
            180             185             190

Ser Gly Ala Phe Lys Cys Leu Gln Asp Gly Ala Gly Asp Val Ala Phe
        195             200             205

Val Lys Glu Thr Thr Val Phe Glu Asn Leu Pro Glu Lys Ala Asp Arg
    210             215             220

Asp Gln Tyr Glu Leu Leu Cys Leu Asn Asn Ser Arg Ala Pro Val Asp
225             230             235             240

Ala Phe Lys Glu Cys His Leu Ala Gln Val Pro Ser His Ala Val Val
        245             250             255

Ala Arg Ser Val Asp Gly Lys Glu Asp Leu Ile Trp Lys Leu Leu Ser
        260             265             270
```

22

```
            Lys Ala Gln Glu Lys Phe Gly Lys Asn Lys Ser Arg Ser Phe Gln Leu
                    275                 280                 285

            Phe Gly Ser Pro Pro Gly Gln Arg Asp Leu Leu Phe Lys Asp Ser Ala
                    290                 295                 300

            Leu Gly Phe Leu Arg Ile Pro Ser Lys Val Asp Ser Ala Leu Tyr Leu
            305                 310                 315                 320

            Gly Ser Arg Tyr Leu Thr Thr Leu Lys Asn Leu Arg Glu Thr Ala Glu
                            325                 330                 335

            Glu Val Lys Ala Arg
                        340
```

```
            <210>  8
            <211>  14
            <212>  PRT
            <213>  Artificial Sequence

            <220>
            <223>  bovine lactoferrin peptide from 441 aa to 454 aa

            <400>  8
```

Lys Ala Asn Glu Gly Leu Thr Trp Asn Ser Leu Lys Asp Lys

1 5 10

```
<210> 9
<211> 23
<212> PRT
<213> Artificial Sequence

<220>
<223> peptide of bovine lactoferrin C-lobe from aa 478 to aa 501

<400> 9
```

```
            Thr Gly Ser Cys Ala Phe Asp Glu Phe Phe Ser Gln Ser Cys Ala Pro
            1                 5                 10                  15

            Gly Ala Asp Pro Lys Ser Arg
                        20
```

```
<210> 10
<211> 12
<212> PRT
<213> Artificial sequence

<220>
<223> bovine lactoferrin C-lobe peptide from aa 619 to aa 630

<400> 10
```

```
            Gly Lys Asn Gly Lys Asn Cys Pro Asp Lys Phe Cys
            1               5               10
```

<210> 11
<211> 6
<212> PRT
<213> Artificial Sequence

<220>
<223> bovine lactoferrin C-lobe peptide from aa 633 to aa 637

<400> 11

```
                    Lys Ser Glu Thr Lys Asn
                    1               5
```

<210> 12
<211> 18
<212> PRT
<213> Artificial sequence

<220>
<223> bovine lactoferrin C-lobe peptide from aa 642 to aa 659

<400> 12

```
        Asn Asp Asn Thr Glu Cys Leu Ala Lys Leu Gly Gly Arg Pro Thr Tyr
        1               5               10                  15

        Glu Glu
```

<210> 13
<211> 5
<212> PRT
<213> Artificial sequence

<220>
<223> bovine lactoferrin C-lobe peptide from aa 425 to aa 429

<400> 13

```
                    Cys Val Leu Arg Pro
                    1               5
```

<210> 14
<211> 4
<212> PRT
<213> Artificial sequence

<220>
<223> bovine lactoferrin C-lobe peptide from aa 426 to aa 429

<400> 14

```
Val Leu Arg Pro
 1
```

<210> 15
<211> 11
<212> PRT
<213> Artificial Sequence

<220>
<223> bovine lactoferrin C-lobe peptide from aa 649 to aa 659

<400> 15

```
Ala Lys Leu Gly Gly Arg Pro Thr Tyr Glu Glu
 1               5                   10
```